# EUROPEAN PATENT APPLICATION

(11) **EP 4 591 870 A1**
(43) Date of publication of application: **30.07.2025**
(21) Application number: 23868253.8
(22) Date of filing: 21.09.2023
(51) Int. Cl.: A61K 35/74, A61P 17/00, C12Q 1/689

(54) **ANTIBACTERIAL COMPOSITION AND METHOD FOR DETERMINING ADMINISTRATION OF SAID ANTIBACTERIAL COMPOSITION TO SUBJECT**

(30) Priority: 22.09.2022 JP 2022151298
(71) Applicant: JSR Corporation, Tokyo 105-8640 (JP); Keio University, Tokyo, 108-8345 (JP)
(72) Inventor: MASUDA Kanae, Tokyo 105-8640 (JP); AOTO Yoshimasa, Tokyo 105-8640 (JP); ISAYAMA Jun, Tokyo 105-8640 (JP); ISHIKAWA Hidefumi, Tokyo 105-8640 (JP); GOJI Hiroshi, Tokyo 105-8640 (JP); WATANABE Kazuto, Tokyo 105-8640 (JP); KAWASAKI Hiroshi, Tokyo 160-8582 (JP); ITO Yoshihiro, Tokyo 160-8582 (JP); AMAGAI Masayuki, Tokyo 160-8582 (JP)
(74) Representative: Dehns
(86) International application number: PCT/JP2023/034260
(87) International publication number: WO 2024/063132

(57) **Abstract**

Provided are an antibacterial composition including bacteria exhibiting antibacterial properties against bacteria positively correlated with a value of SCORAD indicating the severity of atopic dermatitis, and a method for determining administration of the antibacterial composition to a subject. The present invention includes an antibacterial composition including skin resident bacteria as an active ingredient, the antibacterial composition being targeted to inflammation-inducing bacteria (excluding the skin resident bacteria).

## Description

### Technical Field

The present invention relates to an antibacterial composition and a method for determining administration of the antibacterial composition to a subject.

### Background Art

Atopic dermatitis or atopic eczema is a disease of which the major lesion is pruritic eczema and in which exacerbation and remission are repeated. Various etiologies are complexly involved in the pathogenesis of atopic dermatitis on the basis of hypersensitivity of organs including skin caused by, for example, atopic predisposition and fragility of the barrier function.

According to the Japanese Dermatological Association, the three basic items of (1) pruritus, (2) characteristic skin rash, and (3) chronic and recurrent progression are set as diagnostic criteria for atopic dermatitis, and those satisfying these basic items are diagnosed as atopic dermatitis regardless of the severity of symptoms.

In order to select an appropriate treatment for atopic dermatitis, it is necessary to correctly evaluate the severity thereof. One of the severity classification methods for which statistical reliability and validity have been verified is Severity Scoring of Atopic Dermatitis (SCORAD). SCORAD can be calculated, for example, by a method described in Guidelines for the Management of Atopic Dermatitis 2021 (https://www.jsaweb.jp/huge/atopic_gl2021.pdf) of the Japanese Society of Allergology.

Examples of a method for treating atopic dermatitis include, based on the disease state, (a) a drug therapy, (b) an external application therapy/skin care for physiological abnormality of skin, and (c) search for and countermeasures against exacerbating factors, as basic methods.

As a drug therapy, a steroid medicine for external application has been frequently used as a drug for relieving inflammation in atopic dermatitis. When a steroid medicine for external application is used, it is necessary to consider, for example, a dosage form depending on the characteristics and site of the lesion and to select an appropriate steroid medicine for external application depending on the severity. However, although the steroid medicine for external application is a basic drug for atopic dermatitis, it has been pointed out that systemic or local side effects are likely to occur due to, for example, the long-term use of a high rank steroid medicine for external application and the use in a state where the skin barrier is deteriorated.

Under these circumstances, studies have been conducted focusing on Staphylococcus aureus (S. aureus), which is considered to be involved in the onset of atopic dermatitis and exacerbation of inflammation.

For example, Patent Literature 1 discloses a skin resident bacteria balance improver containing, as an active ingredient, lanolin fatty acid or a salt thereof which has a proliferative effect on Staphylococcus epidermidis and an antibacterial effect on Staphylococcus aureus.

On the other hand, Non Patent Literature 1 discloses a bacteriotherapy against S. aureus using Staphylococcus hominis (S. hominis) isolated from the skin of a healthy individual.

### Citation List

### Patent Literature

Patent Literature 1: JP 2021-161034 A

### Non Patent Literature

Non Patent Literature 1: Development of a human skin commensal microbe for bacteriotherapy of atopic dermatitis and use in a phase 1 randomized clinical trial, Nakatsuji et. al., Nature Medicine 27, 700-709 (2021)

### Summary of Invention

### Technical Problem

Since lanolin fatty acid, which is an active ingredient of the skin resident bacteria balance improver of Patent Literature 1, may cause allergy depending on the subject, there is still room for improvement.

Non Patent Literature 1 shows that S. hominis has an antibacterial activity against S. aureus and that S. hominis is safe, but the skin microbiota of patients with atopic dermatitis was diverse, and there was room for improvement from a viewpoint of bacteriotherapy that has taken the severity of atopic dermatitis into consideration.

In view of the above circumstances, an object of the present invention is to provide an antibacterial composition including bacteria exhibiting antibacterial properties against bacteria positively correlated with a value of SCORAD indicating the severity of atopic dermatitis, and a method for determining administration of the antibacterial composition to a subject.

### Solution to Problem

The present invention includes the following [1] to [21].
[1] An antibacterial composition including skin resident bacteria as an active ingredient, the antibacterial composition being targeted to inflammation-inducing bacteria (excluding the skin resident bacteria).
[2] The antibacterial composition according to [1], in which the skin resident bacteria include one or more bacteria selected from the group consisting of bacteria having 16S rDNA containing any one of base sequences set forth in SEQ ID Nos: 1 to 6 or a base sequence having 95% or more identity with any of the base sequences.
[3] The antibacterial composition according to [1] or [2], in which the skin resident bacteria include two or more bacteria selected from the group consisting of bacteria having 16S rDNA containing any one of the base sequences set forth in SEQ ID Nos: 1 to 6 or a base sequence having 95% or more identity with the base sequences.
[4] The antibacterial composition according to any one of [1] to [3], in which the skin resident bacteria include one or more bacteria selected from the group consisting of bacteria having 16S rDNA containing any one of the base sequences set forth in SEQ ID Nos: 1 to 3 or a base sequence having 95% or more identity with the base sequences.
[5] The antibacterial composition according to any one of [1] to [4], in which the skin resident bacteria include two or more selected from the group consisting of bacteria having 16S rDNA containing any one of the base sequences set forth in SEQ ID Nos: 1 to 3 or a base sequence having 95% or more identity with the base sequences.
[6] The antibacterial composition according to any one of [1] to [5], in which the skin resident bacteria include bacteria having 16S rDNA containing the base sequence set forth in SEQ ID No: 1 or a base sequence having 95% or more identity with the base sequence, bacteria having 16S rDNA containing the base sequence set forth in SEQ ID No: 2 or a base sequence having 95% or more identity with the base sequence, and bacteria having 16S rDNA containing the base sequence set forth in SEQ ID No: 3 or a base sequence having 95% or more identity with the base sequence.
[7] The antibacterial composition according to any one of [2] to [6], further including one or more bacteria selected from the group consisting of bacteria having 16S rDNA containing any one of base sequences set forth in SEQ ID Nos: 7 and 8 or a base sequence having 95% or more identity with the base sequences.
[8] The antibacterial composition according to [1], in which the skin resident bacteria include one or more bacteria selected from the group consisting of bacteria having 16S rDNA containing any one of base sequences set forth in SEQ ID Nos: 1 to 13 or a base sequence having 95% or more identity with the base sequences.
[9] The antibacterial composition according to any one of [1] to [8], in which the inflammation-inducing bacteria include one or more bacteria selected from the group consisting of bacteria having 16S rDNA containing any one of base sequences set forth in SEQ ID Nos: 14 and 15 or a base sequence having 95% or more identity with the base sequences.
[10] The antibacterial composition according to [1], in which the skin resident bacteria include one or more bacteria selected from the group consisting of Actionomyces viscosus, Cutibacterium granulosum, Streptococcus oralis, Staphylococcus warneri, Cutibacterium acnes, and Corynebacterium tuberculostearicum.
[11] The antibacterial composition according to [1] or [10], in which the skin resident bacteria include two or more bacteria selected from the group consisting of Actionomyces viscosus, Cutibacterium granulosum, Streptococcus oralis, Staphylococcus warneri, Cutibacterium acnes, and Corynebacterium tuberculostearicum.
[12] The antibacterial composition according to [1] or any one of [10] and [11], in which the skin resident bacteria include one or more bacteria selected from the group consisting of Actionomyces viscosus, Cutibacterium granulosum, and Streptococcus oralis.
[13] The antibacterial composition according to [1] or any one of [10] to [12], in which the skin resident bacteria include two or more bacteria selected from the group consisting of Actionomyces viscosus, Cutibacterium granulosum, and Streptococcus oralis.
[14] The antibacterial composition according to [1] or any one of [10] to [13], in which the skin resident bacteria include Actionomyces viscosus, Cutibacterium granulosum, and Streptococcus oralis.
[15] The antibacterial composition according to any one of [10] to [14], in which the skin resident bacteria further include one or more bacteria selected from the group consisting of Staphylococcus capitis and Staphylococcus epidermidis.
[16] The antibacterial composition according to [1], in which the skin resident bacteria include one or more bacteria selected from the group consisting of Actionomyces viscosus, Cutibacterium granulosum, Streptococcus oralis, Staphylococcus warneri, Cutibacterium acnes, Corynebacterium tuberculostearicum, Staphylococcus capitis, Staphylococcus epidermidis, Moraxella osloensis, Dermacoccus nishinomiyaensis, Micrococcus luteus, Enhydrobacter aerosaccus, and Finegoldia magna.
[17] The antibacterial composition according to [1] or any one of [10] to [16], in which the inflammation-inducing bacteria include one or more bacteria selected from the group consisting of Staphylococcus aureus and Staphylococcus hominis.
[18] The antibacterial composition according to any one of [1] to [17], which is used for treatment or prevention of a disease or condition caused by the inflammation-inducing bacteria.
[19] The antibacterial composition according to [18], in which the disease or condition is one or more diseases or conditions selected from the group consisting of atopic dermatitis, impetigo contagiosa, staphylococcal scalded skin syndrome, purulent mastitis, furunculosis, acne vulgaris, rosacea, contact dermatitis, seborrheic dermatitis, epidermolytic ichthyosis, superficial skin infections, harlequin ichthyosis, congenital ichthyosiform erythroderma, lamellar ichthyosis, Netherton's syndrome, Sjogren-Larsson syndrome, keratitis-ichtyosis-deafness (KID) syndrome, Dorfman-Chanarin syndrome, neutral lipid storage disease, multiple sulfatase deficiency, and X-linked ichthyosis.
[20] A method for determining administration of the antibacterial composition according to any one of [1] to [19] to a subject from a composition ratio or an amount of bacteria, the method including: calculating the composition ratio or the amount of bacteria, with respect to skin microbiota in the subject, of a group consisting of one or more bacteria having 16S rDNA containing any one of the base sequences set forth in SEQ ID Nos: 1 to 13 or a base sequence having 95% or more identity with the base sequences and a group consisting of one or more bacteria having 16S rDNA containing any one of the base sequences set forth in SEQ ID Nos: 14 and 15 or a base sequence having 95% or more identity with the base sequences.
[21] The method according to [20], the method satisfying either one or more of requirements (A) and (B):
   (A) in a case where the composition ratio (the group consisting of one or more bacteria having 16S rDNA containing any one of the base sequences set forth in SEQ ID Nos: 1 to 13 or a base sequence having 95% or more identity with the base sequences : the group consisting of one or more bacteria having 16S rDNA containing any one of the base sequences set forth in SEQ ID Nos: 14 and 15 or a base sequence having 95% or more identity with the base sequences) is 0 : 10 to 9 : 1, it is determined that it is preferable to administer the antibacterial composition according to any one of [1] to [19] to the subject, and
   (B) in a case where the amount of bacteria of the group consisting of one or more bacteria having 16S rDNA containing any one of the base sequences set forth in SEQ ID Nos: 1 to 13 or a base sequence having 95% or more identity with the base sequences is 1000000 CFU/cm² or less, and the amount of bacteria of the group consisting of one or more bacteria having 16S rDNA containing any one of the base sequences set forth in SEQ ID Nos: 14 and 15 or a base sequence having 95% or more identity with the base sequences is 10 CFU/cm² or more, it is determined that it is preferable to administer the antibacterial composition according to any one of [1] to [19] to the subject.

### Advantageous Effects of Invention

According to the present invention, it is possible to provide an antibacterial composition including bacteria exhibiting antibacterial properties against bacteria positively correlated with a value of SCORAD indicating severity of atopic dermatitis, and a method for determining administration of the antibacterial composition to a subject.

In addition, since the skin resident bacteria are fixed to the skin, it is possible to reduce the number of repeated administrations as in the case of the antibacterial drug in the related art.

### Brief Description of Drawings

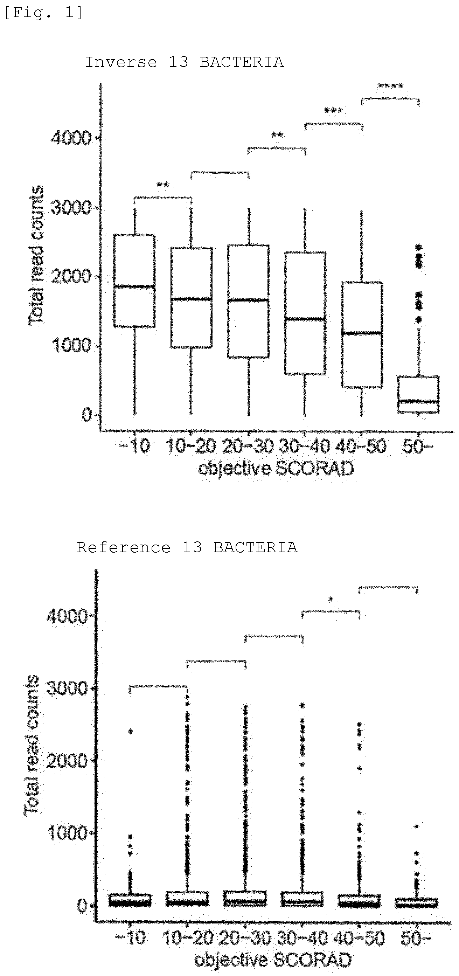

Fig. 1 is a graph illustrating a correlation between 13 species of Inverse bacteria which atopic dermatitis patients have and objective SCORAD and a correlation between 13 species of Reference bacteria and the objectives SCORAD. The vertical axis illustrates total read counts of each bacterial group obtained by 16S rRNA analysis, and the horizontal axis illustrates numerical values of the objective SCORAD.

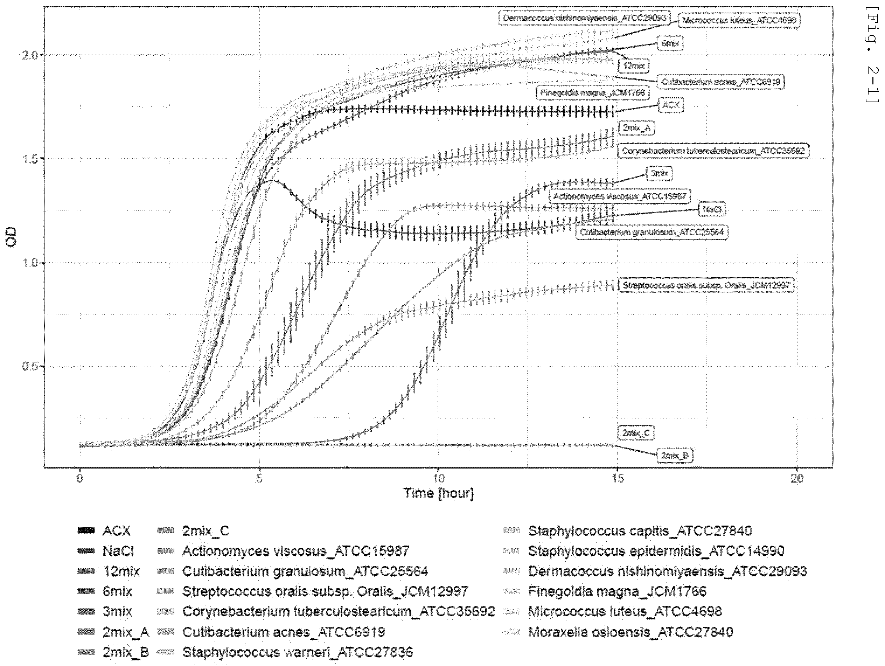

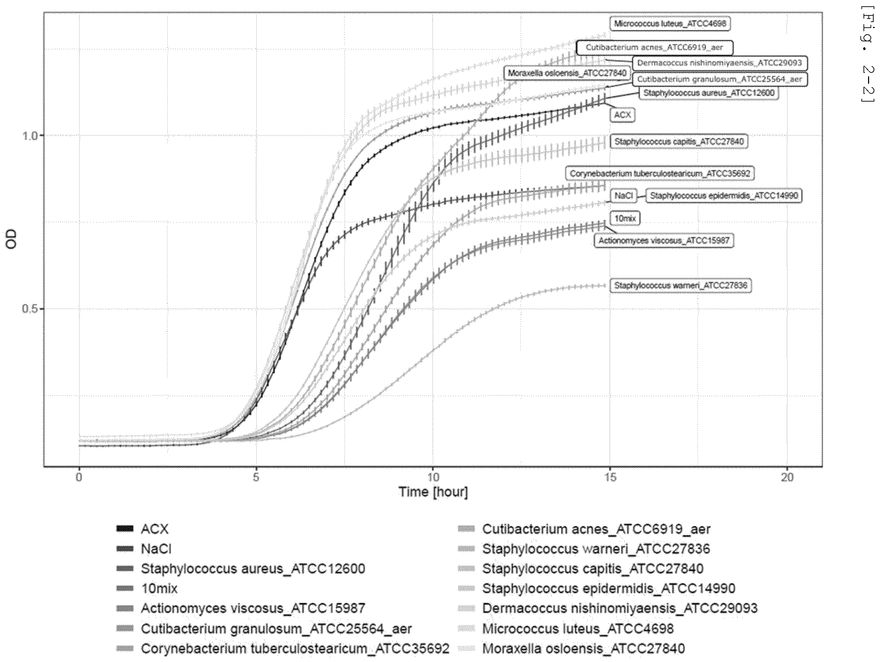

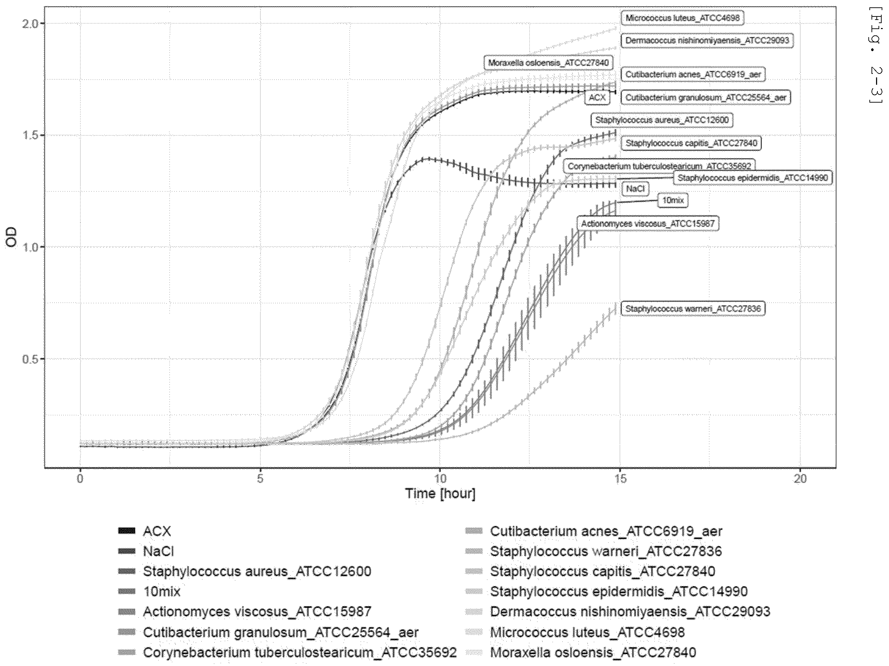

Figs. 2-1 to Fig. 2-3 are graphs illustrating growth curves of S. aureus when S. aureus was cultured using a culture supernatant of Inverse bacteria on different days. The vertical axes illustrate optical density (OD), and the horizontal axes illustrate culture period (hour).

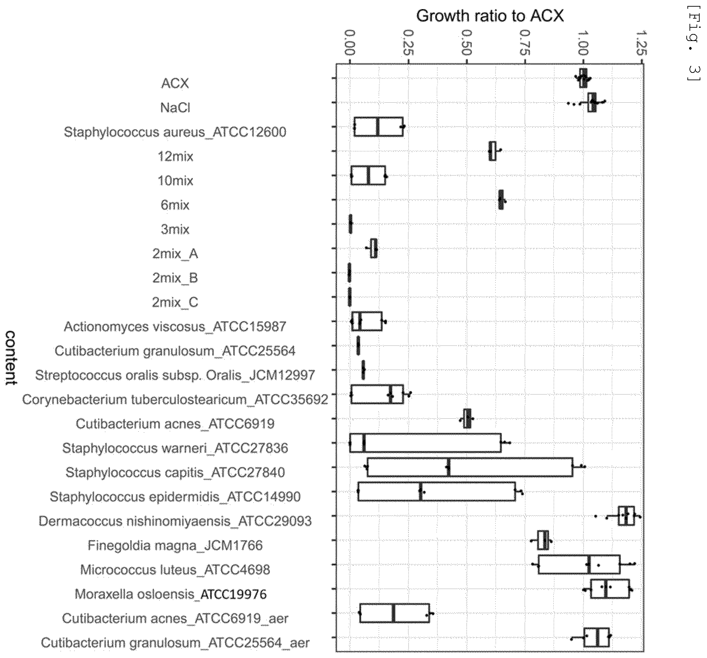

Fig. 3 is a graph summarizing growth inhibitory activity when S. aureus was cultured using a culture supernatant of Inverse bacteria, calculated from results obtained in Figs. 2-1 to 2-3. The vertical axis illustrates a value obtained by normalizing the values of the optical density (OD) at a median time of a logarithmic growth phase, and the horizontal axis illustrates the bacterial species.

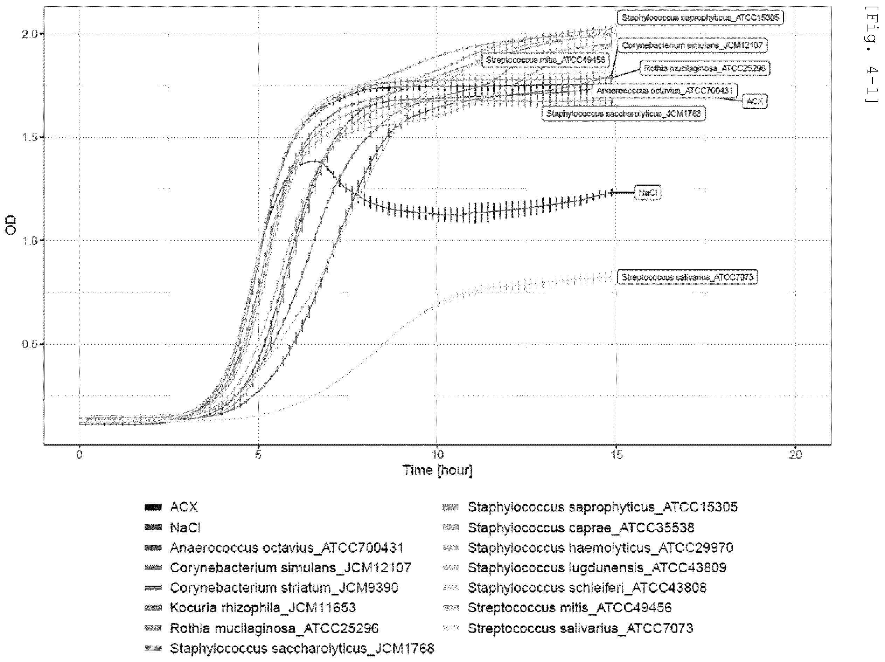

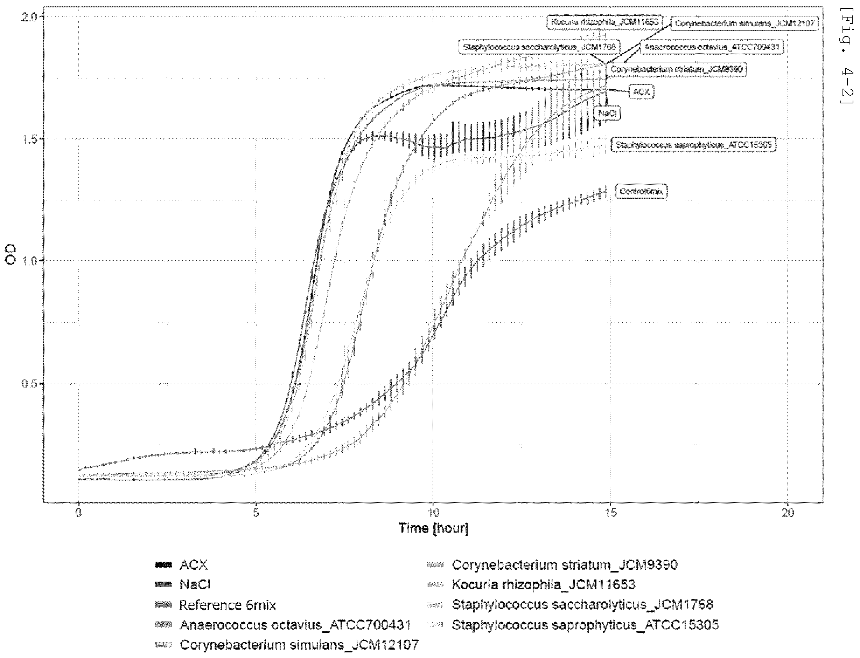

Figs. 4-1 and 4-2 are graphs illustrating growth curves of S. aureus when S. aureus was cultured using a culture supernatant of Reference bacteria on different days. The vertical axes illustrate optical density (OD), and the horizontal axes illustrate culture period (hour).

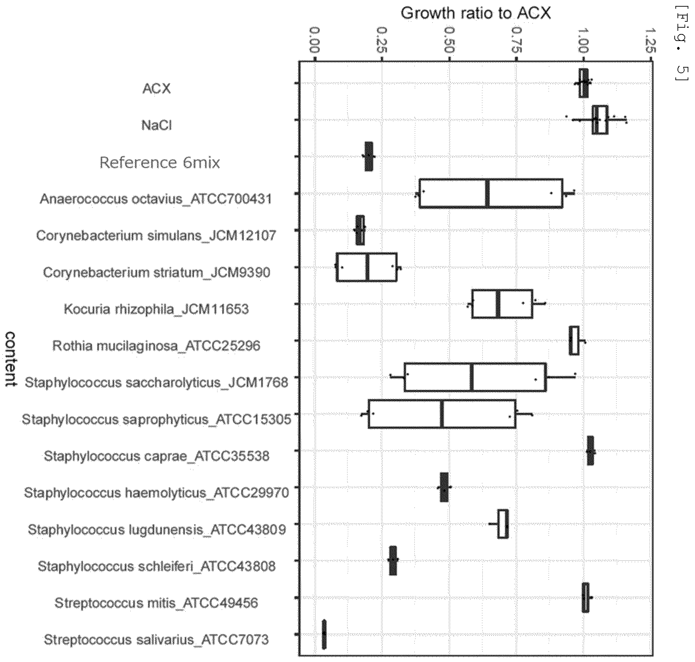

Fig. 5 is a graph summarizing growth inhibitory activity when S. aureus was cultured using a culture supernatant of Reference bacteria, calculated from results obtained in Figs. 4-1 and 4-2. The vertical axis illustrates a value obtained by normalizing the values of the optical density (OD) at a median time of a logarithmic growth phase, and the horizontal axis illustrates the bacterial species.

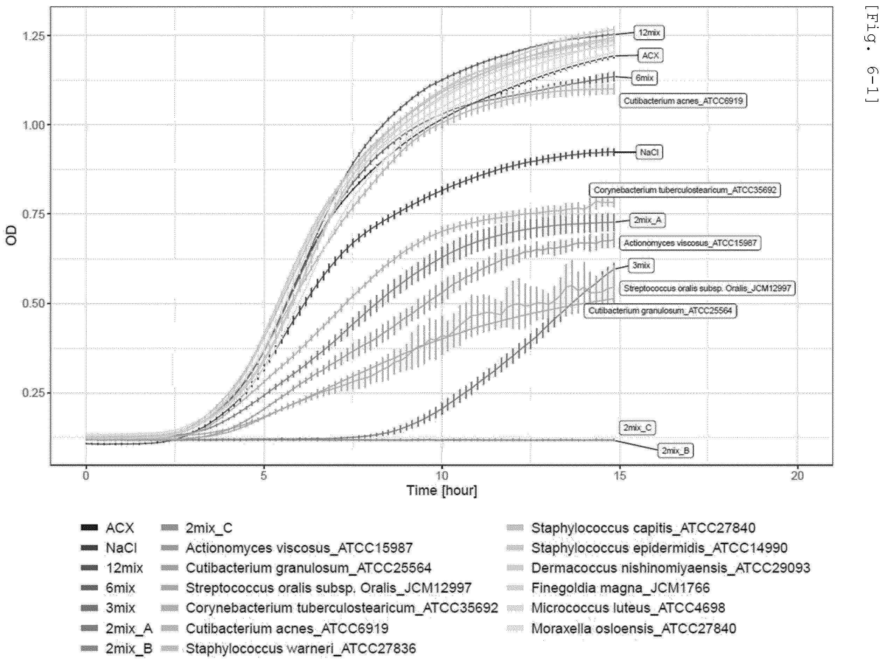

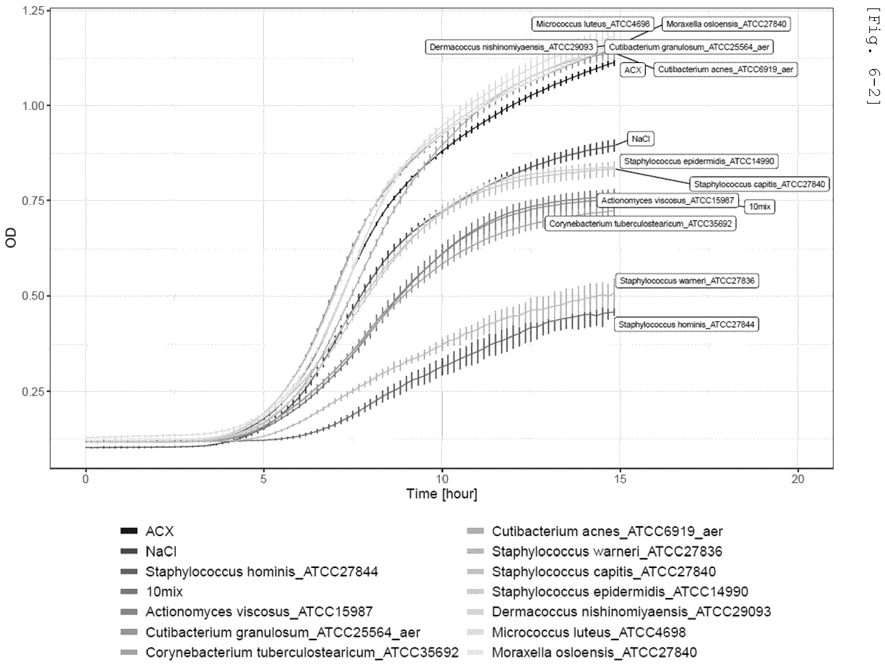

Figs. 6-1 and 6-2 are graphs illustrating growth curves of S. hominis when S. hominis was cultured using a culture supernatant of Inverse bacteria on different days. The vertical axes illustrate optical density (OD), and the horizontal axes illustrate culture period (hour).

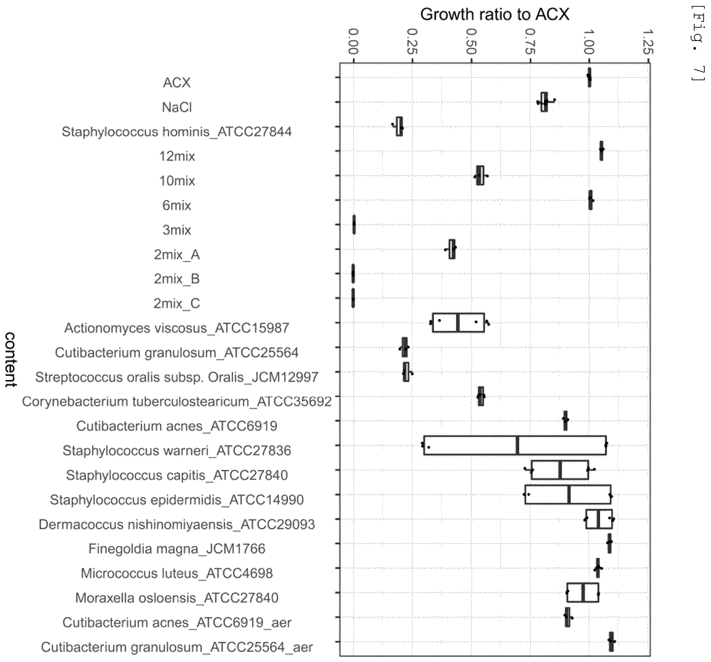

Fig. 7 is a graph summarizing growth inhibitory activity when S. hominis was cultured using a culture supernatant of Inverse bacteria, calculated from results obtained in Figs. 6-1 and 6-2. The vertical axis illustrates a value obtained by normalizing the values of the optical density (OD) at a median time of a logarithmic growth phase, and the horizontal axis illustrates the bacterial species.

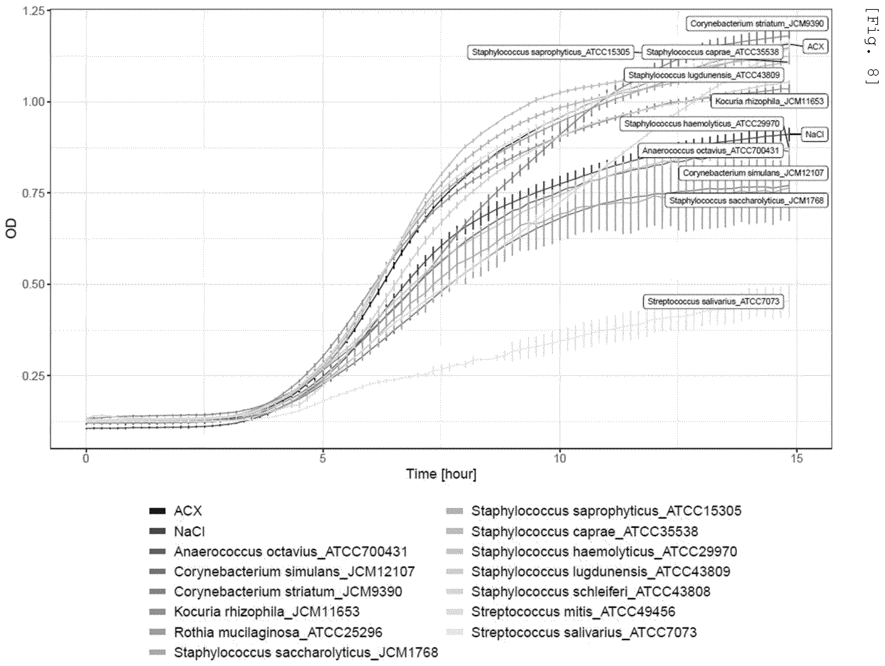

Fig. 8 is a graph illustrating growth curves of S. aureus when S. aureus was cultured using a culture supernatant of Reference bacteria. The vertical axes illustrate optical density (OD), and the horizontal axes illustrate culture period (hour).

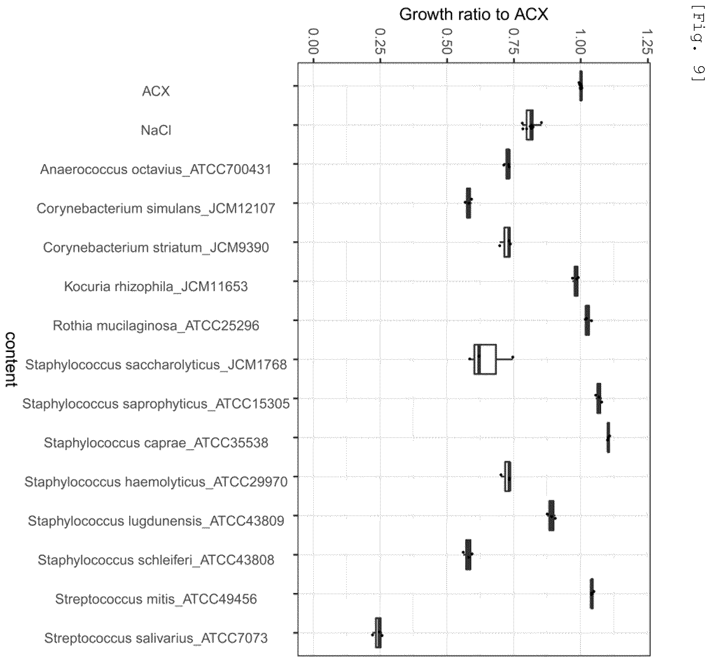

Fig. 9 is a graph illustrating growth inhibitory activity when S. aureus was cultured using a culture supernatant of Reference bacteria, calculated from the result obtained in Fig. 8. The vertical axis illustrates a value obtained by normalizing the values of the optical density (OD) at a median time of a logarithmic growth phase, and the horizontal axis illustrates the bacterial species.

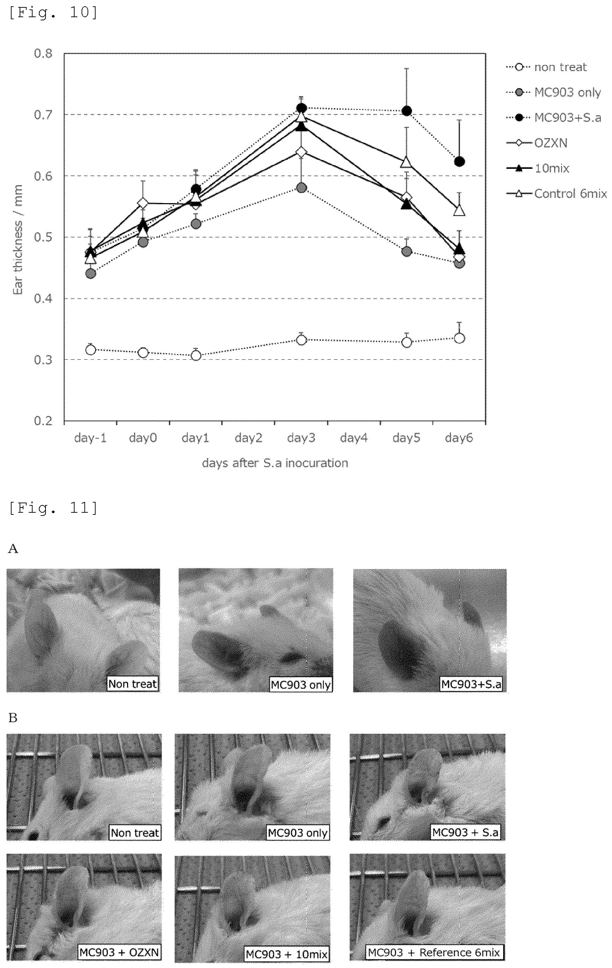

Fig. 10 is a graph illustrating temporal changes in a thickness of auricles when auricles of BALB/cAJcl mice, in which inflammation was induced using MC903, were infected with S. aureus and then treated with ozenoxacin (OZXN) or a culture supernatant of Inverse bacteria (10mix) or Reference bacteria (Reference 6mix).
Fig. 11 is photographs illustrating conditions of ears of mice when auricles of BALB/cAJcl mice, in which inflammation was induced using MC903, were infected with S. aureus and then treated with ozenoxacin (OZXN) or a culture supernatant of Inverse bacteria (10mix) or Reference bacteria (Reference 6mix). A illustrates photographs on Day 0 of the treatment, and B illustrates photographs on Day 6 after the treatment.

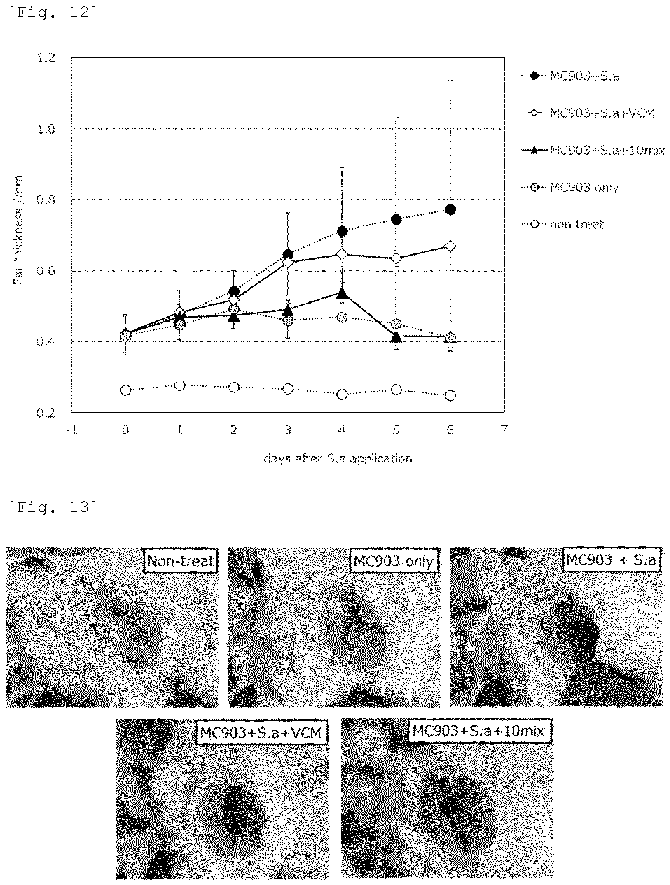

Fig. 12 is a graph illustrating temporal changes in the thickness of auricles when auricles of BALB/cAJcl mice, in which inflammation was induced using MC903, were infected with S. aureus and then treated with vancomycin (VCM) or a culture supernatant of Inverse bacteria (10mix).
Fig. 13 is photographs illustrating conditions of ears of mice on Day 6 after the treatment when auricles of BALB/cAJcl mice, in which inflammation was induced using MC903, were infected with S. aureus and then treated with vancomycin (VCM) or a culture supernatant of Inverse bacteria (10mix).

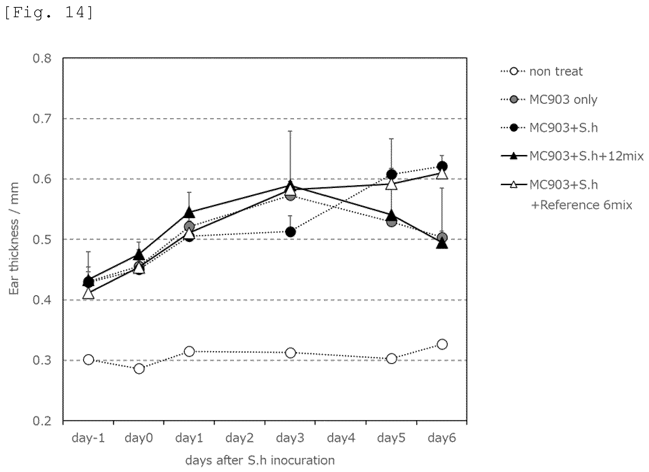

Fig. 14 is a graph illustrating temporal changes in the thickness of auricles when auricles of BALB/cAJcl mice, in which inflammation was induced using MC903, were infected with S. hominis (S. h) and then treated with a culture supernatant of Inverse bacteria (12mix) or Reference bacteria (Reference 6mix).

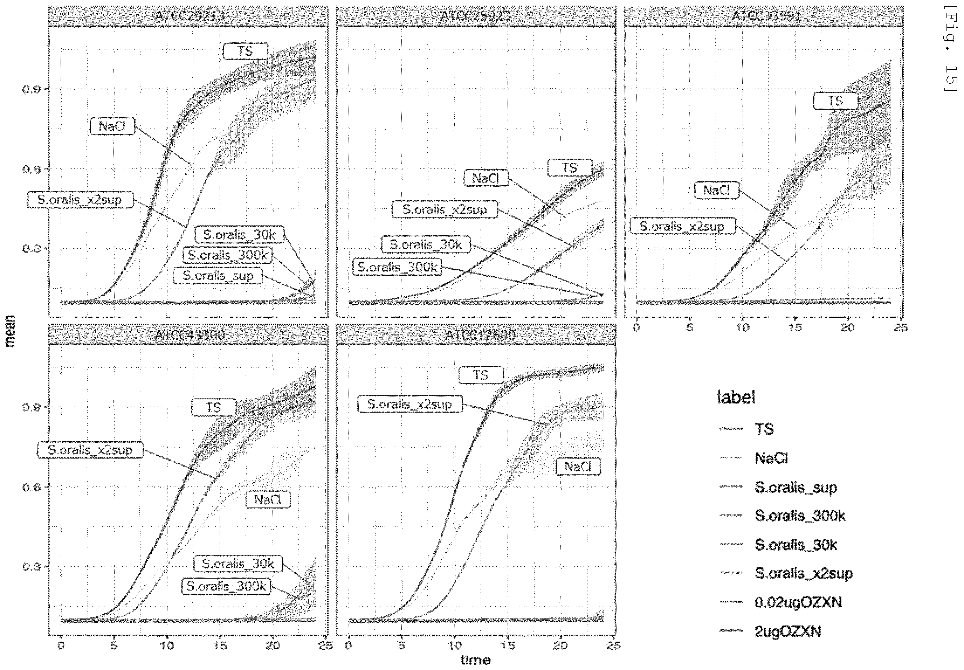

Fig. 15 is graphs illustrating a growth curve of each S. aureus when each S. aureus was cultured using a culture supernatant of S. oralis. The vertical axes illustrate optical density (OD), and the horizontal axes illustrate culture period (hour).

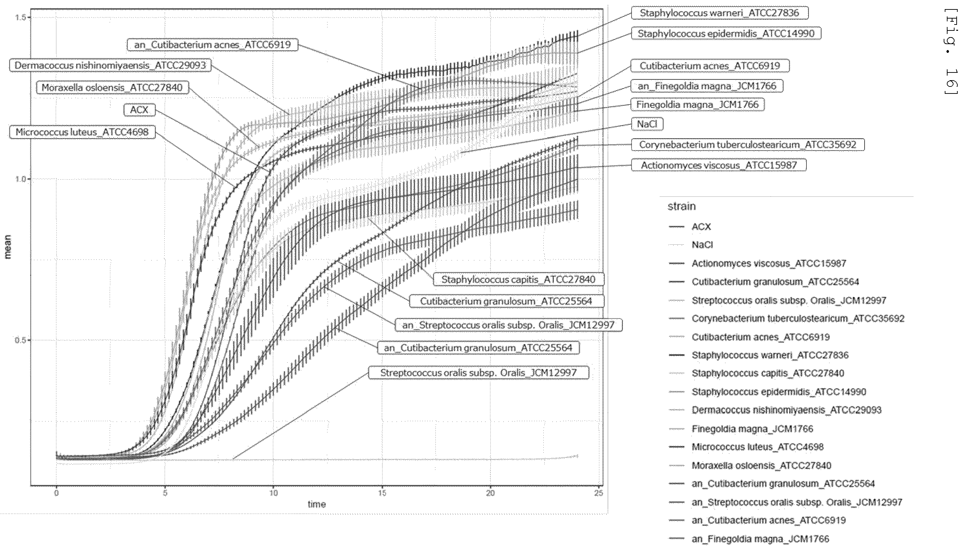

Fig. 16 is a graph summarizing growth inhibitory activity when each S. aureus was cultured using a culture supernatant of S. oralis, calculated from the result obtained in Fig. 15. The vertical axis illustrates a value obtained by normalizing the values of the optical density (OD) at a median time of a logarithmic growth phase, and the horizontal axis illustrates the bacterial species.

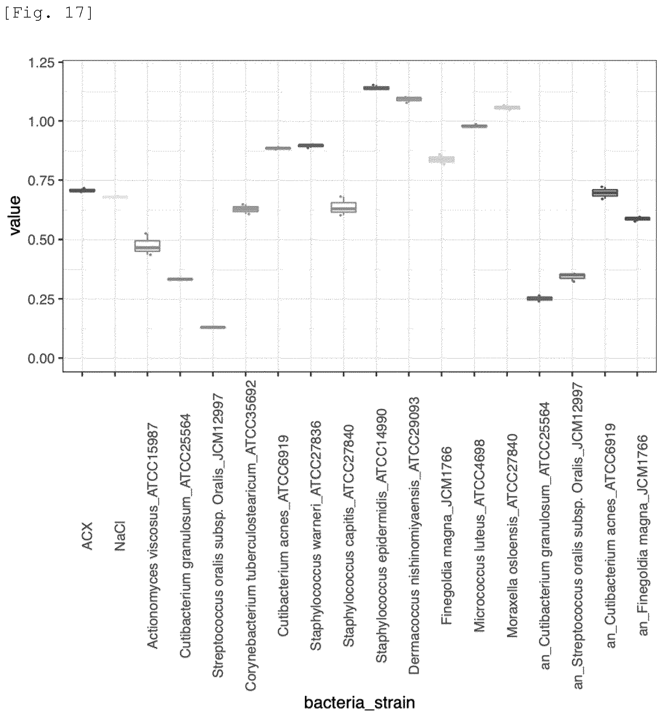

Fig. 17 is a graph illustrating growth curves of S. aureus (MRSA) when S. aureus was cultured using a culture supernatant of Inverse bacteria. The vertical axes illustrate optical density (OD), and the horizontal axes illustrate culture period (hour).

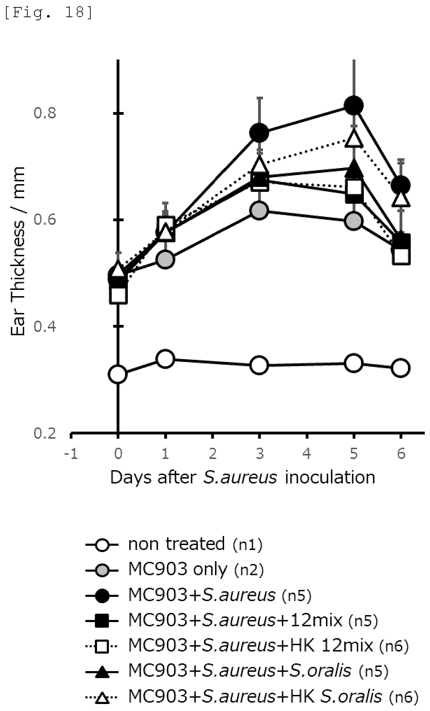

Fig. 18 is a graph illustrating temporal changes in the thickness of auricles when auricles of BALB/cAJcl mice, in which inflammation was induced using MC903, were infected with S. aureus and then treated with any of viable bacteria of Inverse bacteria (12 mix), dead bacteria of Inverse bacteria (12 mix), viable bacteria of S. oralis, and dead bacteria of S. oralis.

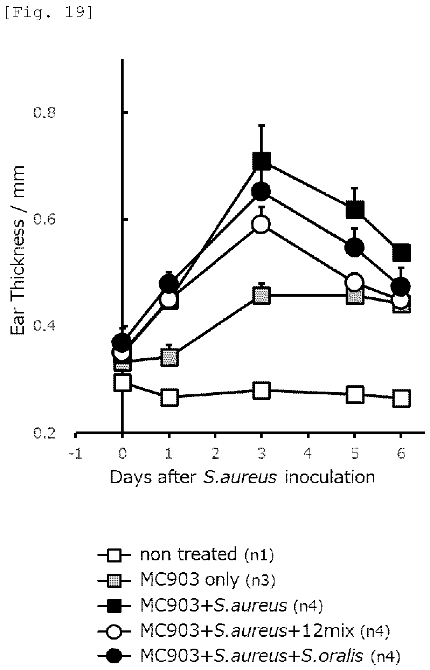

Fig. 19 is a graph illustrating temporal changes in the thickness of auricles when auricles of C57BL/6 mice, in which inflammation was induced using MC903, were infected with S. aureus and then treated with a culture supernatant of Inverse bacteria (12mix) or S. oralis.

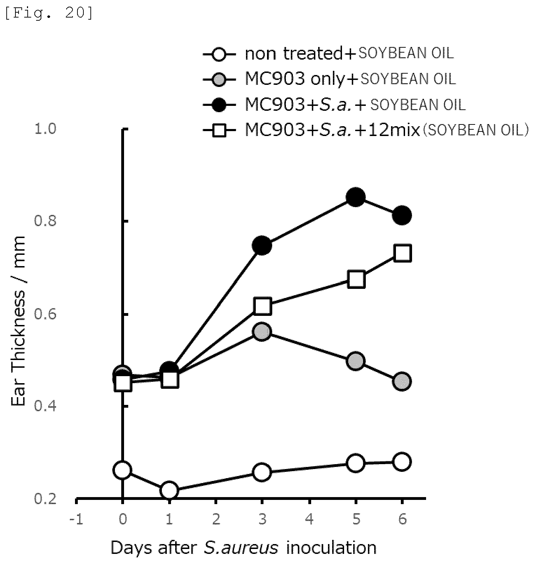

Fig. 20 is a graph illustrating temporal changes in the thickness of auricles when auricles of BALB/cAJcl mice, in which inflammation was induced using MC903, were infected with S. aureus and then treated with Inverse bacteria (12 mix) which were obtained by freeze-drying Inverse bacteria and re-dispersing the bacteria in soybean oil.

### Description of Embodiments

Hereinafter, a preferred embodiment for carrying out the present invention will be described. Note that the embodiment described below is an example of representative embodiments of the present invention, and the scope of the present invention is not narrowly interpreted by this embodiment.

### <Antibacterial Composition>

An embodiment of the present invention is an antibacterial composition including skin resident bacteria as an active ingredient, the antibacterial composition being targeted to inflammation-inducing bacteria (excluding the skin resident bacteria).

The skin resident bacteria included in the antibacterial composition may be viable bacteria or dead bacterial cells. A form of the composition including the skin resident bacteria is not particularly limited, and may be either liquid or solid. In the case of viable bacteria, a form of a culture (a culture supernatant containing viable bacteria and a culture supernatant not containing viable bacteria) obtained after culture, a form obtained by diluting or concentrating the culture, or a form obtained by drying bacterial cells is preferable, and a form of a mixture of these and, for example, a buffer or a base is more preferable. Conditions for culturing viable bacteria are not particularly limited, and aerobic culture or anaerobic culture may be appropriately selected according to the culture stage using a culture medium appropriate for the bacterial species. For example, in the cases of Cutibacterium granulosum, Streptococcus oralis, Cutibacterium acnes, and Finegoldia magna described later in Examples, it is preferable to perform aerobic culture in pre-culture and then perform anaerobic culture in a step of obtaining a culture supernatant from a viewpoint of having a high growth inhibitory effect on the inflammation-inducing bacteria. In the case of dead bacterial cells, a form obtained by drying bacterial cells is preferable, and a form of a mixture of these cells with, for example, a culture medium, a buffer, or a base is more preferable. The drying treatment can be performed, for example, by a method such as heat drying, freeze-drying, or spray drying a suspension obtained by dispersing the bacterial cells in a solvent such as water, and among these, freeze-drying is preferable. In addition, a culture containing dead bacterial cells obtained, for example, by heat-treating a culture of viable bacteria may also be used.

The antibacterial composition may also contain, for example, a substance contained in the skin resident bacteria such as a protein, a nucleic acid, a lipid, a carbohydrate, and a sugar chain, a buffer such as a phosphate, a citrate, and an acetate, a culture medium which can be used for culturing the skin resident bacteria such as sterile water, physiological saline, an ACX culture medium, a clostridium culture medium, a BHI culture medium, a BL culture medium, an LB culture medium, and an EG culture medium, vegetable oil such as soybean oil, rice bran oil, corn oil, olive oil, rapeseed oil, coconut oil, linseed oil, sesame oil, castor oil, and peanut oil, a base such as beeswax, paraffin, liquid paraffin, stearic acid, and white petrolatum, a sugar or a sugar alcohol such as sucrose, lactose, trehalose, dextran, erythritol, arabitol, xylitol, sorbitol, and mannitol; an amino acid such as arginine and histidine; or a stabilizer such as a polyol such as propylene glycol, glycerol, poly(ethylene glycol), and poly(propylene glycol). In addition, when the skin resident bacteria are subjected to freeze-drying treatment, it is particularly preferable to contain glycerol.

### [Skin Resident Bacteria]

The skin resident bacteria included in the antibacterial composition of the present invention is bacteria that exhibit an antibacterial action against the inflammation-inducing bacteria described later by one or combining two or more of bacteria present on the skin of a healthy individual or a patient with a skin disease.

In the present specification, the term "antibacterial" is a concept including sterilization and disinfection, in addition to suppression of proliferation of bacteria. The presence or absence of the antibacterial action can be confirmed by well-known techniques, for example, a method of evaluation from the fluctuation in a growth curve performed in Examples, and a method such as a disk diffusion method and a broth microdilution method.

As the skin resident bacteria, those exhibiting an antibacterial action against the inflammation-inducing bacteria, which are presumed to increase a value of SCORAD (including objective SCORAD in which subjective symptoms are omitted from SCORAD) serving as an index of the severity of atopic dermatitis, are preferable.

For example, in a patient with atopic dermatitis, the composition ratio of skin microbiota before and after treatment such as bleach bath treatment, drug therapies using, for example, an anti-inflammatory medicine for external application and anti-histamine, and ultraviolet treatment is calculated by flora analysis such as 16S rRNA analysis or metagenomic analysis. Next, bacterial species present at a composition ratio of 10% or more, preferably 15% or more, and more preferably 20% or more are selected. Correction is performed by the Benjamini-Hochberg method in single regression with a linear mixed model using the bacterial species as an explanatory variable and SCORAD as an objective variable. If the p-value of the F-test in the same corrected regression is less than 0.1, preferably less than 0.05, more preferably less than 0.01, even more preferably less than 0.001, and particularly preferably less than 0.0001, the inflammation-inducing bacteria can be selected as the bacteria positively correlated with SCORAD, and the bacteria negatively correlated with SCORAD can be selected as the skin resident bacteria.

Examples of the skin resident bacteria include bacteria negatively correlated with SCORAD, for example, bacteria having 16S rDNA containing any one of the base sequences of SEQ ID Nos: 1 to 13 that encode the V1-V2 region of 16S rRNA or a base sequence having identity of 95% or more, preferably 96% or more, more preferably 97% or more, even more preferably 98% or more, and particularly preferably 99% or more with the base sequences. The bacteria may have one or more 16S rDNAs having the base sequences with the same SEQ ID Nos but with different identities. For example, 16S rDNA having the base sequence of SEQ ID **No:** 1 and 16S rDNA having a base sequence having 95% or more identity with SEQ ID No: 1 may be contained in one bacterium. The skin resident bacteria used in Examples described later have 16S rDNA containing any one of the base sequences of SEQ ID Nos: 1 to 13 or a base sequence having 95% or more identity with the base sequences.

As a preferred aspect of the present invention, the antibacterial composition of the present invention includes preferably one or more, more preferably two or more bacteria selected from the group consisting of bacteria having 16S rDNA containing any one of the base sequences set forth in SEQ ID Nos: 1 to 6 or a base sequence having 95% or more, preferably 96% or more, more preferably 97% or more, even more preferably 98% or more, and particularly preferably 99% or more identity with the base sequences.

As a more preferred aspect of the present invention, the antibacterial composition of the present invention includes preferably one or more, more preferably two or more bacteria selected from the group consisting of bacteria having 16S rDNA containing any one of the base sequences set forth in SEQ ID Nos: 1 to 3 or a base sequence having 95% or more, preferably 96% or more, more preferably 97% or more, even more preferably 98% or more, and particularly preferably 99% or more identity with the base sequences.

As an even more preferred aspect of the present invention, the antibacterial composition of the present invention includes bacteria having 16S rDNA containing the base sequence set forth in SEQ ID No: 1 or a base sequence having 95% or more, preferably 96% or more, more preferably 97% or more, even more preferably 98% or more, and particularly preferably 99% or more identity with the base sequence, bacteria having 16S rDNA containing the base sequence set forth in SEQ ID No: 2 or a base sequence having 95% or more, preferably 96% or more, more preferably 97% or more, even more preferably 98% or more, and particularly preferably 99% or more identity with the base sequence, and bacteria having 16S rDNA containing the base sequence set forth in SEQ ID No: 3 or a base sequence having 95% or more, preferably 96% or more, more preferably 97% or more, even more preferably 98% or more, and particularly preferably 99% or more identity with the base sequence.

As a particularly preferred aspect of the present invention, the antibacterial composition of the present invention further includes, in addition to one or more, preferably two or more bacteria having 16S rDNA containing the base sequences set forth in SEQ ID Nos: 1 to 6 or a base sequence having 95% or more, preferably 96% or more, more preferably 97% or more, even more preferably 98% or more, and particularly preferably 99% or more identity with the base sequences, or in addition to one or more, preferably two or more bacteria having 16S rDNA containing the base sequences set forth in SEQ ID Nos: 1 to 3 or a base sequence having 95% or more, preferably 96% or more, more preferably 97% or more, even more preferably 98% or more, and particularly preferably 99% or more identity with the base sequences, one or more bacteria selected from the group consisting of bacteria having 16S rDNA containing any one of the base sequences set forth in SEQ ID Nos: 7 and 8 or a base sequence having 95% or more, preferably 96% or more, more preferably 97% or more, even more preferably 98% or more, and particularly preferably 99% or more identity with the base sequences.

One aspect of the antibacterial composition of the present invention includes one or more bacteria having 16S rDNA containing the base sequences set forth in SEQ ID Nos: 1 to 13 or a base sequence having 95% or more, preferably 96% or more, more preferably 97% or more, even more preferably 98% or more, and particularly preferably 99% or more identity with the base sequences.

When the antibacterial composition of the present invention contains two or more bacteria, for example, 2, 3, 4, 5, 6, 7, 8, 9, 10, 11, 12, or 13 bacteria having 16S rDNA containing any one of the base sequences set forth in SEQ ID Nos: 1 to 13 or a base sequence having 95% or more, preferably 96% or more, more preferably 97% or more, even more preferably 98% or more, and particularly preferably 99% or more identity with the base sequences, the combination of the bacteria can be arbitrarily selected.

In a case where bacteria having 16S rDNA containing any one of the base sequence set forth in SEQ ID No: n or a base sequence having 95% or more identity with the base sequence is abbreviated as "bacteria n", examples of the combination of the bacteria include a combination of bacteria 1 and bacteria 3, a combination of bacteria 2 and bacteria 3, a combination of bacteria 1 and bacteria 3, a combination of bacteria 1, bacteria 2, and bacteria 3, a combination of bacteria 1, bacteria 2, bacteria 3, bacteria 4, bacteria 5, and bacteria 6, a combination of bacteria 1, bacteria 2, bacteria 4, bacteria 5, bacteria 6, bacteria 7, bacteria 8, bacteria 9, bacteria 10, and bacteria 11, a combination of bacteria 1, bacteria 2, bacteria 4, bacteria 5, bacteria 6, bacteria 7, bacteria 8, bacteria 9, bacteria 10, bacteria 11, and bacteria 12, a combination of bacteria 1, bacteria 2, bacteria 3, bacteria 4, bacteria 5, bacteria 6, bacteria 7, bacteria 8, bacteria 9, bacteria 10, bacteria 11, and bacteria 13, a combination of bacteria 1, bacteria 2, bacteria 3, bacteria 4, bacteria 5, bacteria 6, bacteria 7, bacteria 8, bacteria 9, bacteria 10, bacteria 11, bacteria 12, and bacteria 13, a combination of bacteria 3 and bacteria 4, a combination of bacteria 3 and bacteria 5, a combination of bacteria 3 and bacteria 6, a combination of bacteria 3, bacteria 4, and bacteria 7, a combination of bacteria 3, bacteria 4, and bacteria 8, a combination of bacteria 3, bacteria 4, and bacteria 9, a combination of bacteria 3, bacteria 4, and bacteria 10, a combination of bacteria 3, bacteria 4, and bacteria 11, a combination of bacteria 3, bacteria 5, and bacteria 7, a combination of bacteria 3, bacteria 5, and bacteria 8, a combination of bacteria 3, bacteria 5, and bacteria 9, a combination of bacteria 3, bacteria 5, and bacteria 10, a combination of bacteria 3, bacteria 5, and bacteria 11, a combination of bacteria 3, bacteria 6, and bacteria 7, a combination of bacteria 3, bacteria 6, and bacteria 8, a combination of bacteria 3, bacteria 6, and bacteria 9, a combination of bacteria 3, bacteria 6, and bacteria 10, and a combination of bacteria 3, bacteria 6, and bacteria 11.

As the bacteria having 16S rDNA containing any one of the base sequence set forth in SEQ ID No: 1 or a base sequence having 95% or more, preferably 97% or more identity with the base sequence, it is preferable to use Actionomyces viscosus, and the strain number of the bacteria is preferably ATCC15987.

As the bacteria having 16S rDNA containing any one of the base sequence set forth in SEQ ID No: 2 or a base sequence having 95% or more, preferably 97% or more identity with the base sequence, it is preferable to use Cutibacterium granulosum, and the strain number of the bacteria is preferably ATCC25564.

As the bacteria having 16S rDNA containing any one of the base sequence set forth in SEQ ID No: 3 or a base sequence having 95% or more, preferably 97% or more identity with the base sequence, it is preferable to use Streptococcus oralis, and the strain number of the bacteria is preferably JCM12997.

As the bacteria having 16S rDNA containing any one of the base sequence set forth in SEQ ID No: 4 or a base sequence having 95% or more, preferably 97% or more identity with the base sequence, it is preferable to use Staphylococcus warneri, and the strain number of the bacteria is preferably ATCC27836.

As the bacteria having 16S rDNA containing any one of the base sequence set forth in SEQ ID No: 5 or a base sequence having 95% or more, preferably 97% or more identity with the base sequence, it is preferable to use Cutibacterium acnes, and the strain number of the bacteria is preferably ATCC6919.

As the bacteria having 16S rDNA containing any one of the base sequence set forth in SEQ ID No: 6 or a base sequence having 95% or more, preferably 97% or more identity with the base sequence, it is preferable to use Corynebacterium tuberculostearicum, and the strain number of the bacteria is preferably ATCC35692.

As the bacteria having 16S rDNA containing any one of the base sequence set forth in SEQ ID No: 7 or a base sequence having 95% or more, preferably 97% or more identity with the base sequence, it is preferable to use Staphylococcus capitis, and the strain number of the bacteria is preferably ATCC27840.

As the bacteria having 16S rDNA containing any one of the base sequence set forth in SEQ ID No: 8 or a base sequence having 95% or more, preferably 97% or more identity with the base sequence, it is preferable to use Staphylococcus epidermidis, and the strain number of the bacteria is preferably ATCC14990.

As the bacteria having 16S rDNA containing any one of the base sequence set forth in SEQ ID No: 9 or a base sequence having 95% or more, preferably 97% or more identity with the base sequence, it is preferable to use Moraxella osloensis, and the strain number of the bacteria is preferably ATCC27840.

As the bacteria having 16S rDNA containing any one of the base sequence set forth in SEQ ID No: 10 or a base sequence having 95% or more, preferably 97% or more identity with the base sequence, it is preferable to use Dermacoccus nishinomiyaensis, and the strain number of the bacteria is preferably ATCC29093.

As the bacteria having 16S rDNA containing any one of the base sequence set forth in SEQ ID No: 11 or a base sequence having 95% or more, preferably 97% or more identity with the base sequence, it is preferable to use Micrococcus luteus, and the strain number of the bacteria is preferably ATCC4698.

As the bacteria having 16S rDNA containing any one of the base sequence set forth in SEQ ID No: 12 or a base sequence having 95% or more, preferably 97% or more identity with the base sequence, it is preferable to use Enhydrobacter aerosaccus, and the strain number of the bacteria is preferably ATCC27094.

As the bacteria having 16S rDNA containing any one of the base sequence set forth in SEQ ID No: 13 or a base sequence having 95% or more, preferably 97% or more identity with the base sequence, it is preferable to use Finegoldia magna, and the strain number of the bacteria is preferably JCM1766.

As a preferred aspect of the present invention, the antibacterial composition of the present invention includes preferably one or more, and more preferably two or more bacteria selected from the group consisting of Actionomyces viscosus, Cutibacterium granulosum, Streptococcus oralis, Staphylococcus warneri, Cutibacterium acnes, and Corynebacterium tuberculostearicum.

As a more preferred aspect of the present invention, the antibacterial composition of the present invention includes preferably one or more, and more preferably two or more bacteria selected from the group consisting of Actionomyces viscosus, Cutibacterium granulosum, and Streptococcus oralis.

As an even more preferred aspect of the present invention, the antibacterial composition of the present invention includes Actionomyces viscosus, Cutibacterium granulosum, and Streptococcus oralis.

As a particularly preferred aspect of the present invention, the antibacterial composition of the present invention further includes, in addition to one or more bacteria selected from the group consisting of Actionomyces viscosus, Cutibacterium granulosum, Streptococcus oralis, Staphylococcus warneri, Cutibacterium acnes, and Corynebacterium tuberculostearicum, one or more bacteria selected from the group consisting of Staphylococcus capitis and Staphylococcus epidermidis.

One aspect of the antibacterial composition of the present invention includes one or more bacteria selected from the group consisting of Actionomyces viscosus, Cutibacterium granulosum, Streptococcus oralis, Staphylococcus warneri, Cutibacterium acnes, Corynebacterium tuberculostearicum, Staphylococcus capitis, Staphylococcus epidermidis, Moraxella osloensis, Dermacoccus nishinomiyaensis, Micrococcus luteus, Enhydrobacter aerosaccus, and Finegoldia magna.

### [Inflammation-Inducing Bacteria]

The inflammation-inducing bacteria are bacteria that cause inflammation in the skin, which exclude the above-described skin resident bacteria, and include not only bacteria that cause the onset of inflammation but also bacteria that exacerbate inflammation that has already occurred. In addition, these bacteria may be multidrug-resistant bacteria, and may be multidrug-resistant bacteria such as methicillin-resistant Staphylococcus aureus (MRSA), vancomycin-resistant Enterococcus (VRE), penicillin-resistant Streptococcus pneumoniae (PRSP), quinolone-resistant Escherichia coli, carbapenem-resistant Klebsiella pneumoniae, or multidrug-resistant Pseudomonas aeruginosa. Among these multidrug-resistant bacteria, the antibacterial composition of the present invention exhibits a higher effect on methicillin-resistant Staphylococcus aureus (MRSA) and vancomycin-resistant Enterococcus (VRE).

The inflammation-inducing bacteria is not particularly limited as long as they are bacteria that cause inflammation, and bacteria positively correlated with the SCORAD is preferable, and bacteria having 16S rDNA containing any one of the base sequences set forth in SEQ ID Nos: 14 and 15 which encode the V1-V2 region of 16S rRNA or a base sequence having 95% or more, preferably 96% or more, more preferably 97% or more, even more preferably 98% or more, and particularly preferably 99% or more identity with the base sequences is more preferable.

As the bacteria having 16S rDNA containing any one of the base sequence set forth in SEQ ID No: 14 or a base sequence having 95% or more, preferably 97% or more identity with the base sequence, it is preferable to use Staphylococcus aureus, and the strain number of the bacteria is preferably ATCC12600.

As the bacteria having 16S rDNA containing any one of the base sequence set forth in SEQ ID No: 15 or a base sequence having 95% or more, preferably 97% or more identity with the base sequence, it is preferable to use Staphylococcus hominis, and the strain number of the bacteria is preferably ATCC27844.

The inflammation-inducing bacteria may be Staphylococcus aureus with a strain number of ATCC29213 or ATCC25923, or Staphylococcus aureus (Methicillin-resistant Staphylococcus aureus: MRSA) with a strain number of ATCC33591 or ATCC43300.

### [Uses]

The antibacterial composition can be used for the treatment or prevention of a disease or condition caused by the above-described inflammation-inducing bacteria, and may be used as a pharmaceutical composition such as a pharmaceutical product or a quasi-drug, a cosmetic product having a therapeutic or preventive effect on a disease or condition caused by the above-described inflammation-inducing bacteria, or a research application. "Treatment" includes not only complete recovery from the disease or condition, but also alleviation or maintenance of remission of the symptoms of the disease or the condition, or suppression of the progression thereof. "Prevention" includes suppressing or delaying the onset of the disease or the condition and suppressing the recurrence thereof.

The disease or condition caused by the inflammation-inducing bacteria may be any disease or condition as long as it is a disease including dermatitis as a symptom or a condition in which dermatitis is observed even if the name of the disease cannot be determined, and is preferably a disease or condition dependent on S. aureus or S. hominis, more preferably a disease or condition selected from the group consisting of atopic dermatitis, impetigo contagiosa, staphylococcal scalded skin syndrome, purulent mastitis, furunculosis, acne vulgaris, rosacea, contact dermatitis, seborrheic dermatitis, epidermolytic ichthyosis, superficial skin infections, harlequin ichthyosis, congenital ichthyosiform erythroderma, lamellar ichthyosis, Netherton's syndrome, Sjogren-Larsson syndrome, keratitis-ichtyosis-deafness (KID) syndrome, Dorfman-Chanarin syndrome, neutral lipid storage disease, multiple sulfatase deficiency, and X-linked ichthyosis, and even more preferably a disease or condition selected from the group consisting of atopic dermatitis, acne vulgaris, and superficial skin infections.

The antibacterial composition is used by being administered to the skin. The content of the skin resident bacteria in the antibacterial composition is not particularly limited, and can be appropriately set. The dose of the antibacterial composition administered to the subject can be appropriately selected according to, for example, the age, weight, symptom of the disease, and health condition of the subject.

The antibacterial composition can be formulated by a known pharmaceutical method. For example, the antibacterial composition can be formulated as a lotion, a liniment, a cream, an ointment, a plaster, a cataplasm, a spray, or a transdermal medication patch, and among these, it is preferable to formulate the antibacterial composition as an ointment, a cream, a lotion, or a bath additive.

In the formulation, the antibacterial composition can be appropriately combined with, for example, a pharmacologically acceptable carrier, specifically, sterile water, physiological saline, a buffer, a culture medium, vegetable oil, a solvent, a base, an emulsifier, a suspending agent, a surfactant, a stabilizer, a fragrance, an excipient, a vehicle, a preservative, a binder, a diluent, an isotonizing agent, a buffer, a lubricant, a colorant, a thickener, or other additives.

The antibacterial composition can be used for animals including humans, and there is no particular limitation on animals other than humans. The antibacterial composition may be used for, for example, various livestock, poultry, pets, and laboratory animals.

### <Method for Determining Administration of Antibacterial Composition>

An embodiment of the present invention is a method for determining administration of the antibacterial composition of the present invention to a subject from a composition ratio or an amount of bacteria, the method including calculating the composition ratio or the amount of bacteria, with respect to skin microbiota in the subject, of a group consisting of one or more bacteria having 16S rDNA containing any one of the base sequences set forth in SEQ ID Nos: 1 to 13 or a base sequence having 95% or more identity with the base sequences (hereinafter, referred to as "Inverse Mix") and a group consisting of one or more bacteria having 16S rDNA containing any one of the base sequences set forth in SEQ ID Nos: 14 and 15 or a base sequence having 95% or more identity with the base sequences (hereinafter, referred to as "Positive Mix").

The above determination method preferably satisfies either one or more of requirements (A) and (B).
(A) In a case where the composition ratio (the group consisting of one or more bacteria having 16S rDNA containing any one of the base sequences set forth in SEQ ID Nos: 1 to 13 or a base sequence having 95% or more identity with the base sequences : the group consisting of one or more bacteria having 16S rDNA containing any one of the base sequences set forth in SEQ ID Nos: 14 and 15 or a base sequence having 95% or more identity with the base sequences) is 0 : 10 to 9 : 1, it is determined that it is preferable to administer the antibacterial composition to the subject.
(B) In a case where the amount of bacteria of the group consisting of one or more bacteria having 16S rDNA containing any one of the base sequences set forth in SEQ ID Nos: 1 to 13 or a base sequence having 95% or more identity with the base sequences is 1000000 CFU/cm² or less, and the amount of bacteria of the group consisting of one or more bacteria having 16S rDNA containing any one of the base sequences set forth in SEQ ID Nos: 14 and 15 or a base sequence having 95% or more identity with the base sequences is 10 CFU/cm² or more, it is determined that it is preferable to administer the antibacterial composition to the subject.

The above composition ratio can be obtained by flora analysis such as 16S rRNA analysis or metagenomic analysis. When the ratio of each composition ratio (Inverse Mix : Positive Mix) is, for example, 0 : 10 to 9 : 1, 0 : 10 to 8 : 2, 0 : 10 to 7 : 3, 0 : 10 to 6 : 4, 0 : 10 to 5 : 5, 0 : 10 to 4 : 5, 0 : 10 to 3 : 7, 0 : 10 to 2 : 9, or 0 : 10 to 1 : 9, preferably 0 : 10 to 5 : 5, and more preferably 0 : 10 to 3 : 7, it is determined that it is preferable to administer the antibacterial composition of the present invention to the subject. When the ratio of each composition ratio is within the above numerical range, the administration of the antibacterial ingredient suppresses the proliferation of the inflammation-inducing bacteria and has an effect of treating or preventing a disease or condition caused by the inflammation-inducing bacteria.

The respective amounts of bacteria in Inverse Mix and Positive Mix can be determined by, for example, quantitative PCR using known 16S rRNA universal primers. When the amount of bacteria in Inverse Mix is 1000000 CFU/cm² or less, preferably 100000 CFU/cm² or less, more preferably 10000 CFU/cm² or less, and even more preferably 1000 CFU/cm² or less, and the amount of bacteria in Positive Mix is 10 CFU/cm² or more, preferably 100 CFU/cm² or more, more preferably 1000 CFU/cm² or more, and even more preferably 10000 CFU/cm² or more, it is determined that it is preferable to administer the antibacterial composition of the present invention to the subject. When the amounts of bacteria are within the above numerical ranges, the administration of the antibacterial composition suppresses the proliferation of the inflammation-inducing bacteria and has the effect of treating or preventing a disease or condition caused by the inflammation-inducing bacteria. Note that the above determination can be performed with the value of each amount of bacteria of only one of Inverse Mix and Positive Mix, or can be performed with the value of each amount of bacteria of both of Inverse Mix and Positive Mix, but it is preferable to perform the determination with the value of each amount of bacteria of both of Inverse Mix and Positive Mix.

### <Method for Treating or Preventing Disease or Condition Caused by Inflammation-Inducing Bacteria>

An embodiment of the present invention is a method for treating or preventing a disease or condition caused by inflammation-inducing bacteria, the method including a step of calculating a composition ratio or an amount of bacteria, with respect to skin microbiota in a subject, of a group consisting of one or more bacteria having 16S rDNA containing any one of the base sequences set forth in SEQ ID Nos: 1 to 13 or a base sequence having 95% or more identity with the base sequences and a group consisting of one or more bacteria having 16S rDNA containing any one of the base sequences set forth in SEQ ID Nos: 14 and 15 or a base sequence having 95% or more identity with the base sequences, a step of determining administration of an antibacterial composition to a subject from the composition ratio or the amount of bacteria obtained in the above step, and a step of administering the antibacterial composition to the subject in which it is determined that, from the above steps, it is preferable to administer the antibacterial composition.

### <Antibacterial Composition for Treatment or Prevention>

An embodiment of the present invention is an antibacterial composition for use in the treatment or prevention of a disease or condition caused by inflammation-inducing bacteria.

### <Use of Antibacterial Composition for Producing Therapeutic Agent or Prophylactic Agent>

An embodiment of the present invention is a use of an antibacterial composition in the production of a therapeutic agent or prophylactic agent for a disease or condition caused by inflammation-inducing bacteria.

### Examples

Hereinafter, the present invention will be described more specifically based on Examples, but the present invention is not limited to these Examples, and can be appropriately modified without changing the gist of the present invention.

### [Preparation Example]

### [1] Network Analysis

Bacteria positively correlated with objective SCORAD and bacteria negatively correlated with objective SCORAD were selected by the following method.

From 26 atopic dermatitis patients, swab samples of skin flora were obtained at 4 sites (glabella, cubital fossa, inner forearm, and the center of upper back) at a total of 6 time points of 1 month before the start of a 3-month bleach bath treatment, once every month during the treatment, and 1 month after the end of the treatment. The composition ratio at the species level was determined by 16S rRNA analysis of each sample described later. Multiple regression with a linear mixed model was performed on a value obtained by logarithmically transforming read counts of 34 bacterial species present in 25% or more of the samples among all bacterial species with a constant of 2. The linear mixed model is a type of statistical analysis model, and is represented by a formula defined by y = Xβ + Zb + ε. The term Xβ is referred to as a fixed effect, the term Zb is referred to as a random effect, y is referred to as an objective variable, X is referred to as an explanatory variable, and Z is referred to as a random effect variable. The linear mixed model was used as a model for evaluating interlocking between each bacterial species by setting objective SCORAD as the objective variable, and a linear mixed model was performed by setting each single bacterial species as the objective variable. In the linear mixed model with objective SCORAD as the objective variable, 34 bacterial species were used as the explanatory variable, and the patients and the collection sites were used as the random effect. In the linear mixed model using each bacteria as the objective variable, 33 bacterial species other than the target bacterial species were used as the explanatory variables, and the patients and the collection sites were used as the random effect. The p-value of the F-test in the regression was corrected by the Benjamini-Hochberg method, and it was defined that there was a correlation between the variables with p-value < 0.0001. Two species of bacteria positively correlated with objective SCORAD (hereinafter also referred to as "Positive bacteria"), 13 species of bacteria negatively correlated with the two bacteria (hereinafter also referred to as "Inverse bacteria"), and 13 species of control bacteria of Inverse bacteria (skin resident bacteria other than Positive bacteria and Inverse bacteria) (hereinafter also referred to as "Control bacteria" or "Reference bacteria") were selected. As the 13 species of Reference bacteria, the top 13 species by the average relative abundance ratio were selected among the skin resident bacteria other than Positive bacteria and Inverse bacteria in the samples. Names of the bacterial species and strain numbers are shown in Table 1. Regarding the names of the bacterial species corresponding to the strain numbers in Table 1, in a case where the scientific name of the bacterial species is changed due to, for example, a change in classification accompanying progress in taxonomic research, the name refers to the bacterial species of the changed scientific name.

**[Table 1]**

| | Name of bacterial species | Strain number |
|---|---|---|
| Positive bacteria | *Staphylococcus aureus* | ATCC12600 |
| | *Staphylococcus hominis* | ATCC27844 |
| | *Staphylococcus warneri* | ATCC27836 |
| | *Actinomyces viscosus* | ATCC15987 |
| | *Cutibacterium acnes* | ATCC6919 |
| | *Staphylococcus capitis* | ATCC27840 |
| Inverse bacteria | *Staphylococcus epidermidis* | ATCC14990 |
| | *Corynebacterium tuberculostearicum* | ATCC35692 |
| | *Moraxella osloensis* | ATCC27840 |
| | *Dermacoccus nishinomiyaensis* | ATCC29093 |
| | *Micrococcus luteus* | ATCC4698 |
| | *Cutibacterium granulosum* | ATCC25564 |
| | *Enhydrobacter aerosaccus* | ATCC27094 |
| | *Finegoldia magna* | JCM 1766 |
| | *Streptococcus oralis* | JCM12997 |
| | *Anaerococcus octavius* | ATCC700431 |
| | *Corynebacterium simulans* | JCM 12107 |
| | *Corynebacterium striatum* | JCM9390 |
| | *Kocuria rhizophila* | JCM 11653 |
| | *Rothia mucilaginosa* | ATCC25296 |
| | *Staphylococcus captrae* | ATCC35538 |
| Reference bacteria | *Staphylococcus haemolyticus* | ATCC29970 |
| | *Staphylococcus lugdunensis* | ATCC43809 |
| | *Staphylococcus saccharolyticus* | JCM 1768 |
| | *Staphylococcus saprophyticus* | ATCC15305 |
| | *Staphylococcus schleiferi* | ATCC43808 |
| | *Streptococcus mitis* | ATCC49456 |
| | *Streptococcus salivarius* | ATCC7073 |

The correlation between the 13 species of Inverse bacteria of the atopic dermatitis patients and objective SCORAD and the correlation between the 13 species of Reference bacteria and objective SCORAD from the results of the 16S rRNA analysis before the treatment of the bleach bath treatment are shown in Fig. 1.

### [2] 16S rRNA Analysis

The 16S rRNA analysis was performed as follows. The bacterial cells in the swab samples obtained from the glabella, cubital fossa, inner forearm, and the center of upper back of the atopic dermatitis patients were suspended in sterile water and then centrifuged (3,000 x g, 4 minutes, room temperature) to collect the bacterial cells. Genomic DNA of all bacterial cells was extracted from the obtained bacterial cells using Maxwell (registered trademark) RSC Blood DNA Kit [AS1400] (manufactured by Promega Corporation). Using the obtained genomic DNA as a template, PCR was performed using the following primers. The temperatures and cycle conditions for the PCR are as follows.

(a) 2 minutes at 96°C
(b) 30 seconds at 96°C
(c) 45 seconds at 55°C
(d) 60 seconds at 72°C
   30 cycles of (b) to (d)
(e) 10 minutes at 72°C
(f) Maintained at 4°C

Fw primer: AGRGTTTGATYMTGGCTCAG (SEQ ID No: 16)
Rv primer: TGCTGCCTCCCGTAGGAGT (SEQ ID No: 17)

DNA fragments of about 300 bp encoding the V1-V2 region of the amplified 16S rRNA were purified using AMPure (manufactured by Beckman Coulter, Inc.). The obtained amplified fragments (amplicons) were subjected to analysis by next generation sequencing (NGS). NGS was performed using Miseq and Miseq Ragent Kit v2 (500-cycle, manufactured by Illumina, Inc.). The two paired end reads were subjected to primer sequence removal using cutadapt, and then filtering was performed using filterAndTrim of dada2 R package (version 1.14.1) under the conditions of maxEE = c(2, 2) and truncLen = c(200, 180). The pair end reads denoised using dada were merged, and chimera removal was performed by removeBimeraDenovo to create an Amplicon Sequence Variant (ASV) table. The ASV table was normalized to 3000 reads per sample by random extraction. The taxonomic assignment of each ASV was performed by searching homology with the Ribosomal Database Project (RDP) (rekaese 11) and National Center for Biotechnology Information (NCBI) genome database by the GLSEARCH program. Note that the present search and assignment, for example, were performed on June 18, 2019. The relationship between the name of each bacterial species and SEQ ID No indicating the sequence of the amplified fragment of each bacterial species is shown in Table 2 below.

**[Table 2]**

| Name of bacterial species | SEQ ID No. |
|---|---|
| *Actinomyces viscosus* | 1 |
| *Cutibacterium granulosum* | 2 |
| *Streptococcus oralis* | 3 |
| *Staphylococcus warneri* | 4 |
| *Cutibacterium acnes* | 5 |
| *Corynebacterium tuberculostearicum* | 6 |
| *Staphylococcus capitis* | 7 |
| *Staphylococcus epidermidis* | 8 |
| *Moraxella osloensis* | 9 |
| *Dermacoccus nishinomiyaensis* | 10 |
| *Micrococcus luteus* | 11 |
| *Enhydrobacter aerosaccus* | 12 |
| *Finegoldia magna* | 13 |
| *Staphylococcus aureus* | 14 |
| *Staphylococcus hominis* | 15 |

### [Example 1]

Each of the species of Inverse bacteria in Table 1 precultured at 37°C for 24 hours in an ACX liquid culture medium (39.5 g/L Blood Agar Basal Culture Medium No. 2 (manufactured by Lab MIDG), 3 g/L yeast extract (manufactured by Oxoid Limited), 2 g/L glucose, 5 mL/L Tween 80, and 50 mL/L defibrinated horse blood (manufactured by TSC Biosciences Ltd.)) was diluted in the ACX liquid culture medium to obtain a single bacterial solution having an optical density (OD) of 0.05 at a wavelength of 600 nm. Each of the obtained single bacterial solutions was cultured at 37°C for 48 hours. The culture solution was centrifuged (10000 x g, 5 minutes) and filtered through a 0.22-um filter to obtain a culture supernatant.

S. aureus precultured in the ACX liquid culture medium was diluted in the ACX liquid culture medium to obtain a Positive bacterial solution having an OD of 0.01. Equal amounts of the Positive bacterial solution and the culture supernatant obtained above were mixed together, then inoculated on a 96 well culture plate (product name "Violamo 96 Well Plate Flat-Bottom", manufactured by Violamo), and cultured at 37°C. In addition, as a comparative control group of the culture supernatant to be mixed, the ACX liquid culture medium and physiological saline not subjected to the culture were used. The OD of the bacterial solution in the culture process was monitored using a plate reader (product name "Microplate Reader Spark", manufactured by Tecan Group Ltd.) to obtain a growth curve. This operation was performed 3 times, each on different days, and the results of the growth curves for the single bacterial solutions are each shown in Figs. 2-1 to 2-3.

The median time of the logarithmic growth phase under a culture condition in which an equal amount of the ACX liquid culture medium was mixed, which was the comparative control group, was defined as the evaluation time, and the OD value at the evaluation time was evaluated as an index of the growth inhibitory activity of the culture supernatant of each Inverse bacteria on Positive bacteria. The result of the growth inhibitory activity of each single bacterial solution is shown in Fig. 3.

### [Example 2]

A culture supernatant of each mix bacterial solution was obtained in the same manner as in Example 1, except that each mix bacterial solution of Inverse bacteria obtained by mixing and combining an equal amount of each bacterial species shown in the following Table 3 was used instead of the single bacterial solutions in Example 1. The results of the growth curves for the mix solutions are each shown in Figs. 2-1 to 2-3, and the results of summarizing the values obtained by normalizing the optical density (OD) values at the median time of the logarithmic growth phase in the growth curves of Figs. 2-1 to 2-3 as growth inhibitory activity are shown in Fig. 3.

**[Table 3]**

| Name of bacterial species | 12mix | 10mix | 6mix | 3mix | 2mix-A | 2mix-B | 2mix-C |
|---|---|---|---|---|---|---|---|
| *Actinomyces viscosus* | ○ | ○ | ○ | ○ | ○ | ○ | |
| *Cutibacterium granulosum* | ○ | ○ | ○ | ○ | ○ | | ○ |
| *Streptococcus oralis* | ○ | | ○ | ○ | | ○ | ○ |
| *Corynebacterium tuberculostearicum* | ○ | ○ | ○ | | | | |
| *Cutibacterium acnes* | ○ | ○ | ○ | | | | |
| *Staphylococcus warneri* | ○ | ○ | ○ | | | | |
| *Staphylococcus capitis* | ○ | ○ | | | | | |
| *Staphylococcus epidermidis* | ○ | ○ | | | | | |
| *Dermacoccus nishinomiyaensis* | ○ | ○ | | | | | |
| *Finegoldia magna* | ○ | | | | | | |
| *Micrococcus luteus* | ○ | ○ | | | | | |
| *Moraxella osloensis* | ○ | ○ | | | | | |

### [Reference Example 1]

A growth curve for each of the single bacterial solutions was obtained in the same manner as in Example 1, except that Reference bacteria were used instead of Inverse bacteria in Table 1 in Example 1. This operation was performed twice, each on different days, and the results of the growth curves of the single bacterial solutions are each shown in Figs. 4-1 and 4-2, and the results of growth inhibitory activity are shown in Fig. 5.

### [Reference Example 2]

Culture supernatants of mix bacterial solutions were obtained in the same manner as in Example 2, except that mix bacterial solutions (Reference 6mix) of Reference bacteria obtained by mixing and combining equal amounts of single bacterial solutions of each of six species of Reference bacteria (Anaerococcus octavius, Corynebacterium simulans, Corynebacterium striatum, Kocuria rhizophila, Staphylococcus saccharolyticus, and Staphylococcus saprophyticus) were used instead of the mix bacterial solution of Inverse bacteria, and the operation was performed twice, each on different days, in Example 2. The results of the growth curves for the mix solutions are shown in Figs. 4-1 and 4-2, and the results of the growth inhibitory activity are shown in Fig. 5.

### [Example 3]

A growth curve of each single bacterial solution was obtained in the same manner as in Example 1, except that S. hominis was used instead of S. aureus, and the operation was performed twice, each on different days, in Example 1. The results of the growth curves for the single bacterial solutions are each shown in Figs. 6-1 and 6-2, and the results of the growth inhibitory activity are shown in Fig. 7.

### [Example 4]

A culture supernatant of each mix bacterial solution was obtained in the same manner as in Example 2, except that S. hominis was used instead of S. aureus, and the operation was performed twice, each on different days, in Example 2. The results of the growth curves for the mix bacterial solutions are each shown in Figs. 6-1 and 6-2, and the results of the growth inhibitory activity are shown in Fig. 7.

### [Reference Example 3]

A growth curve for each of the single bacterial solutions was obtained in the same manner as in Example 1, except that Reference bacteria were used instead of Inverse bacteria in Table 1, and the operation was performed once in Example 3. The results of the growth curves for the single bacterial solutions are each shown in Fig. 8, and the results of the growth inhibitory activity are shown in Fig. 9.

### [Reference Example 4]

A growth curve of each single bacterial solution was obtained in the same manner as in Example 4, except that mix bacterial solutions (Reference 6mix) of Reference bacteria obtained by mixing and combining equal amounts of single bacterial solutions of each of six species of Reference bacteria (Anaerococcus octavius, Corynebacterium simulans, Corynebacterium striatum, Kocuria rhizophila, Staphylococcus saccharolyticus, and Staphylococcus saprophyticus) were used instead of the mix bacterial solution of Inverse bacteria, and the operation was performed once in Example 4. The results of the growth curves for the single bacterial solutions are each shown in Fig. 8, and the results of the growth inhibitory activity are shown in Fig. 9.

### [Example 5]

### [1] Preparation of Mice

BALB/cAJcl mice (8 weeks old, male, manufactured by CLEA Japan, Inc.) were acclimatized for 1 week under controlled temperature and humidity (22 to 24°C, 40 to 60%) and modulated light environment (200 lux/12-hour cycles of day and night switching), and subjected to a comparative experiment on the following 5 groups of (a) to (e).

(a) Untreated group
(b) Group treated with only MC903
(c) Group treated with MC903 + S. aureus
(d) Group treated with MC903 + S. aureus + antibiotic
(e) Group treated with MC903 + S. aureus + bacteria mix

### [2] Creation of MC903-Induced Inflammation Model

A 100 µM ethanol solution of calcipotriol (product name "MC903", manufactured by Funakoshi Co., Ltd.) was applied to both ears of each of the mice in groups (b) to (e) in an amount of 20 µL using a micropipette so as to spread over the entire auricles. An MC903-induced inflammation model was created by applying the solution once a day for one week.

### [3] S. aureus Infection

S. aureus (hereinafter, also referred to as "S. a") was cultured at 37°C in an ACX liquid culture medium to obtain a bacterial solution in a stationary phase of growth. This bacterial solution was diluted in the ACX liquid culture medium to adjust the OD to 2.5. 50 µL of the diluted bacterial solution was applied to both ears of each of the mice in groups (c) to (e) in which inflammation was induced by MC903 using a micropipette so as to spread over the entire auricles. S. aureus was applied only once, and the day on which S. aureus was applied was defined as DAY 0, and every day elapsed was defined as DAY 1, DAY 2, and so on.

### [4] Application of Antibiotic

50 µL of ozenoxacin (ABX) (product name: "Zebiax Lotion", manufactured by Maruho Co., Ltd.) was applied to both ears of the mice in group (d) infected with S. aureus using a micropipette so as to spread over the entire auricles. The application was performed once a day from DAY 1 to DAY 4.

### [5] Application of Bacterial Mix Solution

Each strain of 10mix in Table 3 or each strain of the 6 species of Reference bacteria (Anaerococcus octavius, Corynebacterium simulans, Corynebacterium striatum, Kocuria rhizophila, Staphylococcus saccharolyticus, and Staphylococcus saprophyticus) precultured in the ACX liquid culture medium was diluted in the ACX liquid culture medium, and the solutions of the strains having the same OD values were mixed in equal amounts to obtain 10mix and Reference 6mix, and then glycerol was further added to obtain 20 vol% glycerol stock solutions. Each of the stock solutions was cryopreserved at -80°C and thawed before application. The thawed bacterial stock solution was centrifuged (5,000 x g, 5 minutes), the supernatant was removed, and then re-dispersion was performed in the ACX liquid culture medium to obtain a bacterial mix solution having an OD of 2.5. 50 µL of the bacterial mix solution was applied to both ears of the mice in group (e) infected with S. aureus using a micropipette so as to spread over the entire auricles. The application was performed once a day from DAY 1 to DAY 4. In addition, for group (c) infected with S. aureus, an ACX culture medium not containing bacterial cells was applied in the same manner.

### [6] Measurement of Ear Thickness

Three measurement points were determined on the auricle per one ear, a total of six points on the right and left were measured with a digital caliper, and the average value thereof was taken as the thickness of the auricle. A thickness of the auricle was used as an index of the degree of inflammation in the skin, and the degree of suppression of the inflammation by the treatment with the bacterial mix solution was evaluated. In addition, the ears of the mice were photographed. The ear thicknesses were measured every day from the day before DAY 0 (DAY -1) to DAY 8. The photographing was performed on DAY 0 and DAY 6. The results are shown in Figs. 10 and 11.

### [Example 6]

The degree of suppression of inflammation by the treatment with the bacterial mix solution was evaluated in the same manner as in Example 5, except that the following "[4'] Administration of Antibiotic" was performed instead of "[4] Application of Antibiotic" in Example 5. The results are shown in Figs. 12 and 13. Note that, regarding the conditions of the auricles 0 day after the treatment, the conditions shown in Fig. 11A can be referred to.

### [4'] Administration of Antibiotic

Group (d) infected with S. aureus was intraperitoneally administered with 200 µL of an 18 mg/mL PBS solution of vancomycin (VCM) (manufactured by TOWA PHARMACEUTICAL CO., LTD.). The administration was performed twice a day for 4 days from DAY 1 to DAY 4.

### [Example 7]

The degree of suppression of inflammation by the treatment with the bacterial mix solution was evaluated in the same manner as in Example 5, except that S. hominis (hereinafter, also referred to as "S. h") was used instead of S. a in the section of "[3] S. aureus Infection", and each strain of 12mix was used instead of each strain of 10mix in Table 3 in Example 5. The results are shown in Fig. 14.

### [Example 8]

Growth curves were obtained and the growth inhibitory activity was evaluated in the same manner as in Example 1, except that each of S. aureus shown in the following Table 4 was used as Positive bacteria instead of S. aureus (ATCC12600), and S. oralis (JSM12997) was used as Inverse bacteria in Example 1. The results of the growth curves for each of the single bacterial solutions are shown in Fig. 15, and the growth inhibitory activities are shown in Fig. 16. In Fig. 15, "300K" and "30K" indicate that the supernatant was passed through a filter having a molecular weight cutoff of 300000 or 30000, respectively, and "× 2sup" indicates that the supernatant was subjected to 2-fold dilution.

**[Table 4]**

| Strain number | Name of bacterial species |
|---|---|
| ATCC29213 | S.aureus |
| ATCC25923 | S.aureus |
| ATCC33591 | S. aureus (MRSA) |
| ATCC43300 | S. aureus (MRSA) |
| ATCC12600 | S.aureus |

### [Example 9]

Growth curves were obtained in the same manner as in Example 1, except that S. aureus (ATCC33591) which is MRSA was used as Positive bacteria, and the bacteria shown in Table 1 were used as Inverse bacteria in Example 1. The results of the growth curves of the single bacterial solutions are each shown in Fig. 17. In addition, growth curves of S. aureus in culture supernatants obtained by performing anaerobic culture (the same operation as in Example 1 was performed in Bactron 300 (manufactured by Shellab) which is an anaerobic chamber) on 4 species of bacteria among Inverse bacteria without performing the aerobic culture in Example 1 were also obtained. In the drawing, the names of the bacterial species subjected to the anaerobic culture were given a prefix "an".

### [Example 10]

The degree of suppression of inflammation by the treatment with the bacterial mix solution or S. oralis solution was evaluated in the same manner as in Example 5, except that the following "[5'] Application of Bacterial Mix Solution" was performed instead of "[5] Application of Bacterial Mix Solution" in Example 5. The results are shown in Fig. 18.

### [5]' Application of Bacterial Mix Solution

Each of the strains precultured in the ACX liquid culture medium was diluted in the ACX liquid culture medium, and the diluted strains having the same OD value were mixed in equal amounts to obtain 12mix, and then glycerol was further added to obtain 20 vol% glycerol stock solutions. Each of the stock solutions was cryopreserved at -80°C and thawed before application. After centrifuging the thawed bacterial stock solutions (5,000 x g, 5 minutes) and removing the supernatants, heat treatment was performed at 121°C for 10 minutes, and then re-dispersion was performed in the ACX liquid culture medium, thus preparing Heat Killing (HK) 12mix solutions. Similarly, heat treatment was also performed on S. oralis to prepare a HK S. oralis solution.

### [Example 11]

The degree of suppression of inflammation by the treatment with the bacterial mix solution was evaluated in the same manner as in Example 5, except that the following "[1'] Preparation of Mice" was performed instead of "[1] Preparation of Mice" in Example 5. The results are shown in Fig. 19.

### [1'] Preparation of Mice

C57BL/6 mice (6 to 8 weeks old, male, manufactured by CLEA Japan, Inc.) were acclimatized for 1 week under controlled temperature and humidity (22 to 24°C, 40 to 60%) and modulated light environment (200 lux/12-hour cycles of day and night switching), and subjected to a comparative experiment on the following 5 groups of (a') to (e').

(a') Untreated group
(b') Group treated with only MC903
(c') Group treated with MC903 + S. aureus
(d') Group treated with MC903 + S. aureus + bacteria mix
(e') Group treated with MC903 + S. aureus + S. oralis

### [Example 12]

The degree of suppression of inflammation by the treatment with the bacterial mix solution or S. oralis solution was evaluated in the same manner as in Example 5, except that the following "[5"] Application of Bacterial Mix Solution" was performed instead of "[5] Application of Bacterial Mix Solution" in Example 5. The results are shown in Fig. 20.

### [5"] Application of Bacterial Mix Solution

### (1) Method for Culturing Bacteria

12 kinds of bacteria which had been cryopreserved in the ACX culture medium having the composition shown in the following Table 5 were caused to be dormant, and inoculation and culture were started under the conditions shown in the following Table 6. After about 24 hours, OD = 0.01/50 mL was prepared and passaging was performed, and passaging was further performed in the same manner after about 24 hours. Passaging was performed twice since the bacteria became dormant, and then the bacteria were subjected to freeze-drying.

**[Table 5]**

| Reagent | Manufacturer | Model No. | Amount mixed |
|---|---|---|---|
| Proteose Peptone No.3 | Gibco | 211693 | 15 g |
| Yeast Extract | Becton Dickinson | 212750 | 8 g |
| NaCl | Fujifilm Wako Pure Chemical | 191-01665 | 5g |
| Liver Digest Neutralized | Oxoid | LP0027 | 2.5 g |
| D(+)-Glucose | Fujifilm Wako Pure Chemical | 049-31165 | 2g |
| Tween (registered trademark) 80 | Nacalai | 35703-75 | 5ml |
| MilliQ water | | | 1000ml |

### [Table 6]

**Table 6**

| No. | Bacterial species | Culture condition | | | | |
|---|---|---|---|---|---|---|
| | | Culture condition | Atmosphere | Temperature | Stirring method | Shaking speed (rpm) |
| 47 | *Staphylococcus warneri* | | Aerobic | | Shaking | 200 |
| 48 | *Staphylococcus epidermidis* | | | | | |
| 49 | *Moraxella osloensis* | | | | | |
| 50 | *Dermacoccus nishinomiyaensis* | | | | | |
| 51 | *Corynebacterium tuberculostearicum* | ACX | | 37°C | | |
| 52 | *Staphylococcus capitis* | | | | | |
| 54 | *Micrococcus luteus* | | | | | |
| 55 | *Actinomyces viscosus* | | | | | |
| 56 | *Cutibacterium acnes* | | Anaerobic | | Standing | |
| 57 | *Cutibacterium granulosum* | | | | | |
| 58 | *Streptococcus oralis* | | | | | |
| 45 | *Finegoldia magna* | | | | | |

**Table 6 (continued)**

| No. | Bacterial species | Restoration culture culture/Pl | Passage culture/P2 | | Passage culture/P3 | |
|---|---|---|---|---|---|---|
| | | Inoculation amount (*µ* l)/culture medium 5 mL | OD | Passaging and inoculation amount (*µ* l)/medium 50 mL | OD | Passaging and inoculation amount (*µ* l)/medium 50 mL |
| 47 | *Staphylococcus warneri* | | 3.21 | 156 | 8.41 | 59 |
| 48 | *Staphylococcus epidermidis* | | 3.99 | 125 | 3.79 | 132 |
| 49 | *Moraxella osloensis* | | 1.61 | 311 | 0.86 | 581 |
| 50 | *Dermacoccus nishinomiyaensis* | | 1.66 | 301 | 6.92 | 72 |
| 51 | *Corynebacterium tuberculostearicum* | | 2.68 | 187 | 0.90 | 556 |
| 52 | *Staphylococcus capitis* | 50 | 4.24 | 118 | 8.88 | 56 |
| 54 | *Micrococcus luteus* | | 2.47 | 202 | 9.98 | 50 |
| 55 | *Actinomyces viscosus* | | 2.06 | 301 | 0.80 | 625 |
| 56 | *Cutibacterium acnes* | | 0.13 | 3,846 | 1.34 | 373 |
| 57 | *Cutibacterium granulosum* | | 0.17 | 2,941 | 0.85 | 588 |
| 58 | *Streptococcus oralis* | | 1.38 | 362 | 1.93 | 259 |
| 45 | *Finegoldia magna* | | 1.03 | 485 | 6.89 | 562 |

### (2) Preparation of Bacterial Mix Solution

12 species of bacteria were weighed in the amounts shown in the following Table 7 and added to 36 mL of soybean oil (manufactured by FUJIFILM Wako Pure Chemical Corporation, model number 190-03776), and stirring was performed. After the stirring, the mixture was passed through a filter having a pore size of 100 µm to obtain a stock solution for a preliminary drug. On the day of application, a base material (soybean oil (manufactured by FUJIFILM Wako Pure Chemical Corporation, model number 190-03776)) of a solution for administration was blended with the stock solution for administration at a volume ratio of 7 : 3, thus obtaining a bacterial mix solution. The preparation was performed so that the viable cell count was 4.0E + 09 cell/mL.

**[Table 7]**

| No. | Name of bacteria | Weighed value |
|---|---|---|
| 47 | *Staphylococcus warneri* | 216.7 mg |
| 48 | *Staphylococcus epidermidis* | 45.4 mg |
| 49 | *Moraxella osloensis* | 46.5 mg |
| 50 | *Dermacoccus nishinomiyaensis* | 446.7 mg |
| 51 | *Corynebacterium tuberculostearicum* | 140.8 mg |
| 52 | *Stanhylococcus capitis* | 40.3 mg |
| 54 | *Micrococcus luteus* | 38.5 mg |
| 55 | *Actinomyces viscosus* | 26.9 mg |
| 56 | *Cutibacterium acnes* | 77.1 mg |
| 57 | *Cutibacterium granulosum* | 56.5 mg |
| 58 | *Streptococcus oralis* | 39.1 mg |
| 45 | *Finegoldia magna* | 68.1 mg |

From the above results, it was shown that the proliferation of S. aureus (including MRSA) and S. hominis, which are the inflammation-inducing bacteria, is suppressed by the composition according to the present invention. In addition, it was shown that the composition according to the present invention suppresses the progression of inflammation in animals in which the inflammation was induced by S. aureus and S. hominis, which are the inflammation-inducing bacteria.

This application claims priority based on Japanese Patent Application No. 2022-151298 filed on September 22, 2022, the entire disclosure of which is incorporated herein.

## Claims

1. An antibacterial composition comprising: skin resident bacteria as an active ingredient, the antibacterial composition being targeted to inflammation-inducing bacteria (excluding the skin resident bacteria).

2. The antibacterial composition according to claim 1, wherein the skin resident bacteria include one or more bacteria selected from the group consisting of bacteria having 16S rDNA containing any one of base sequences set forth in SEQ ID Nos: 1 to 6 or a base sequence having 95% or more identity with any of the base sequences.

3. The antibacterial composition according to claim 1, wherein the skin resident bacteria include two or more bacteria selected from the group consisting of bacteria having 16S rDNA containing any one of base sequences set forth in SEQ ID Nos: 1 to 6 or a base sequence having 95% or more identity with the base sequences.

4. The antibacterial composition according to claim 1, wherein the skin resident bacteria include one or more bacteria selected from the group consisting of bacteria having 16S rDNA containing any one of base sequences set forth in SEQ ID Nos: 1 to 3 or a base sequence having 95% or more identity with the base sequences.

5. The antibacterial composition according to claim 1, wherein the skin resident bacteria include two or more selected from the group consisting of bacteria having 16S rDNA containing any one of base sequences set forth in SEQ ID Nos: 1 to 3 or a base sequence having 95% or more identity with the base sequences.

6. The antibacterial composition according to claim 1, wherein the skin resident bacteria include bacteria having 16S rDNA containing a base sequence set forth in SEQ ID No: 1 or a base sequence having 95% or more identity with the base sequence, bacteria having 16S rDNA containing a base sequence set forth in SEQ ID No: 2 or a base sequence having 95% or more identity with the base sequence, and bacteria having 16S rDNA containing a base sequence set forth in SEQ ID No: 3 or a base sequence having 95% or more identity with the base sequence.

7. The antibacterial composition according to any one of claims 2 to 6, further comprising:
one or more bacteria selected from the group consisting of bacteria having 16S rDNA containing any one of base sequences set forth in SEQ ID Nos: 7 and 8 or a base sequence having 95% or more identity with the base sequences.

8. The antibacterial composition according to claim 1, wherein the skin resident bacteria include one or more bacteria selected from the group consisting of bacteria having 16S rDNA containing any one of base sequences set forth in SEQ ID Nos: 1 to 13 or a base sequence having 95% or more identity with the base sequences.

9. The antibacterial composition according to claim 1, wherein the inflammation-inducing bacteria include one or more bacteria selected from the group consisting of bacteria having 16S rDNA containing any one of base sequences set forth in SEQ ID Nos: 14 and 15 or a base sequence having 95% or more identity with the base sequences.

10. The antibacterial composition according to claim 1, wherein the skin resident bacteria include one or more bacteria selected from the group consisting of Actionomyces viscosus, Cutibacterium granulosum, Streptococcus oralis, Staphylococcus warneri, Cutibacterium acnes, and Corynebacterium tuberculostearicum.

11. The antibacterial composition according to claim 1, wherein the skin resident bacteria include two or more bacteria selected from the group consisting of Actionomyces viscosus, Cutibacterium granulosum, Streptococcus oralis, Staphylococcus warneri, Cutibacterium acnes, and Corynebacterium tuberculostearicum.

12. The antibacterial composition according to claim 1, wherein the skin resident bacteria include one or more bacteria selected from the group consisting of Actionomyces viscosus, Cutibacterium granulosum, and Streptococcus oralis.

13. The antibacterial composition according to claim 1, wherein the skin resident bacteria include two or more bacteria selected from the group consisting of Actionomyces viscosus, Cutibacterium granulosum, and Streptococcus oralis.

14. The antibacterial composition according to claim 1, wherein the skin resident bacteria include Actionomyces viscosus, Cutibacterium granulosum, and Streptococcus oralis.

15. The antibacterial composition according to any one of claims 10 to 14, wherein the skin resident bacteria further include one or more bacteria selected from the group consisting of Staphylococcus capitis and Staphylococcus epidermidis.

16. The antibacterial composition according to claim 1, wherein the skin resident bacteria include one or more bacteria selected from the group consisting of Actionomyces viscosus, Cutibacterium granulosum, Streptococcus oralis, Staphylococcus warneri, Cutibacterium acnes, Corynebacterium tuberculostearicum, Staphylococcus capitis, Staphylococcus epidermidis, Moraxella osloensis, Dermacoccus nishinomiyaensis, Micrococcus luteus, Enhydrobacter aerosaccus, and Finegoldia magna.

17. The antibacterial composition according to claim 1, wherein the inflammation-inducing bacteria include one or more bacteria selected from the group consisting of Staphylococcus aureus and Staphylococcus hominis.

18. The antibacterial composition according to claim 1, which is used for treatment or prevention of a disease or condition caused by the inflammation-inducing bacteria.

19. The antibacterial composition according to claim 18, wherein the disease or condition is one or more diseases or conditions selected from the group consisting of atopic dermatitis, impetigo contagiosa, staphylococcal scalded skin syndrome, purulent mastitis, furunculosis, acne vulgaris, rosacea, contact dermatitis, seborrheic dermatitis, epidermolytic ichthyosis, superficial skin infections, harlequin ichthyosis, congenital ichthyosiform erythroderma, lamellar ichthyosis, Netherton's syndrome, Sjogren-Larsson syndrome, keratitis-ichtyosis-deafness (KID) syndrome, Dorfman-Chanarin syndrome, neutral lipid storage disease, multiple sulfatase deficiency, and X-linked ichthyosis.

20. A method for determining administration of the antibacterial composition according to claim 1 to a subject from a composition ratio or an amount of bacteria, the method comprising: calculating the composition ratio or the amount of bacteria, with respect to skin microbiota in the subject, of a group consisting of one or more bacteria having 16S rDNA containing any one of base sequences set forth in SEQ ID Nos: 1 to 13 or a base sequence having 95% or more identity with the base sequences and a group consisting of one or more bacteria having 16S rDNA containing any one of base sequences set forth in SEQ ID Nos: 14 and 15 or a base sequence having 95% or more identity with the base sequences.

21. The method according to claim 20, the method satisfying either one or more of requirements (A) and (B):
(A) in a case where the composition ratio (the group consisting of one or more bacteria having 16S rDNA containing any one of the base sequences set forth in SEQ ID Nos: 1 to 13 or a base sequence having 95% or more identity with the base sequences : the group consisting of one or more bacteria having 16S rDNA containing any one of the base sequences set forth in SEQ ID Nos: 14 and 15 or a base sequence having 95% or more identity with the base sequences) is 0 : 10 to 9 : 1, it is determined that it is preferable to administer the antibacterial composition according to claim 1 to the subject, and
(B) in a case where the amount of bacteria of the group consisting of one or more bacteria having 16S rDNA containing any one of the base sequences set forth in SEQ ID Nos: 1 to 13 or a base sequence having 95% or more identity with the base sequences is 1000000 CFU/cm² or less, and the amount of bacteria of the group consisting of one or more bacteria having 16S rDNA containing any one of the base sequences set forth in SEQ ID Nos: 14 and 15 or a base sequence having 95% or more identity with the base sequences is 10 CFU/cm² or more, it is determined that it is preferable to administer the antibacterial composition according to claim 1 to the subject.
